(19) **Europäisches Patentamt / European Patent Office / Office européen des brevets**

(11) **EP 4 722 702 A1**

(12) **EUROPEAN PATENT APPLICATION**
published in accordance with Art. 153(4) EPC

(43) Date of publication:
08.04.2026 Bulletin 2026/15

(21) Application number: 24815248.0

(22) Date of filing: 17.05.2024

(51) International Patent Classification (IPC):
*G01N 27/00* (2006.01)   *C12Q 1/68* (2018.01)

(52) Cooperative Patent Classification (CPC):
C12Q 1/68; G01N 27/00

(86) International application number:
PCT/JP2024/018330

(87) International publication number:
WO 2024/247764 (05.12.2024 Gazette 2024/49)

(84) Designated Contracting States:
AL AT BE BG CH CY CZ DE DK EE ES FI FR GB
GR HR HU IE IS IT LI LT LU LV MC ME MK MT NL
NO PL PT RO RS SE SI SK SM TR
Designated Extension States:
BA
Designated Validation States:
GE KH MA MD TN

(30) Priority: 30.05.2023 JP 2023089095

(71) Applicant: The University of Tokyo
Bunkyo-ku, Tokyo 113-8654 (JP)

(72) Inventors:
• SUZUKI, Tsutomu
Tokyo 113-8654 (JP)
• NOGUCHI, Ryo
Tokyo 113-8654 (JP)

(74) Representative: D Young & Co LLP
3 Noble Street
London EC2V 7BQ (GB)

Remarks:
The complete document including Reference
Table(s) and the Sequence Listing(s) can be
downloaded from the EPO website

(54) **MODIFICATION ANALYSIS FOR MOLECULE BY SIGNAL-BASED METHOD**

(57)  The present disclosure provides a novel method and the like for analyzing a modification state of constituent units in a molecule in which a plurality of constituent units of one or more types are bonded together. The present disclosure relates to a method for analyzing a modification state of a constituent unit in a molecule in which a plurality of constituent units of one or more types are bonded together, the method including: acquiring series data of signal values that vary in accordance with a type and a modification state of each constituent unit, the series data being obtained by scanning the molecule for each of a plurality of analyte molecules; aligning each of the plurality of series data, acquired from the plurality of analyte molecules, with reference data, thereby aligning the plurality of series data with each other; and analyzing a modification state of at least a part of the plurality of analyte molecules, based on the aligned plurality of series data.

[Figure 2]

## Description

## Technical Field

The present invention relates to a method for analyzing a modification state of a molecule by a signal-based method, and to an analysis system, an analysis device, a program, and a diagnostic support method related thereto.

## Background Art

Proteins are biosynthesized by translation from mRNA serving as a template. In this process, tRNA functions as an adapter molecule that decodes 61 types of genetic codes (codons) encoded in mRNA into 20 types of amino acids. tRNA matures through diverse chemical modifications (tRNA modifications) after transcription, thereby eliciting normal functionality.

To date, approximately 150 types of RNA modifications have been found in various species, and about 80% of these have been found in tRNA. RNA modifications not only complement the functions of RNA but also play a role in regulating gene expression, with entirely new functions being imparted. It is known that various modifications are formed in and around the anticodon of tRNA, and these tRNA modifications play important roles in accurate codon decoding and in maintaining the reading frame during translation. In addition, many modifications exist in the central portion (body) of tRNA, and these are known to be mainly involved in folding of tRNA and in stability in a cell.

In recent years, it has been found that the modification state of tRNA dynamically changes in accordance with growth environment and various external stresses. In order to correctly understand the functions of tRNA modifications, it is necessary to accurately and rapidly map what types of RNA modifications exist at which positions in individual tRNA molecules. Although mapping by high-performance liquid chromatography-mass spectrometry (LC/MS) enables measurement with high sensitivity, it requires isolation and purification of corresponding RNA, and thus it is difficult to map multiple specimens simultaneously or to perform periodic measurement.

As a next-generation sequencer (NGS), a single-molecule sequencer capable of directly analyzing RNA has been developed. For example, a nanopore sequencer is a single-molecule sequencer that enables direct sequence analysis by measuring a current value (ion current signal) of single-stranded DNA or RNA passing through a nanopore formed of a membrane protein. When a modified base in an RNA strand passes through the nanopore, a characteristic current value different from that of an unmodified base is exhibited, making it possible to detect all modifications simultaneously.

In recent years, research on detecting RNA modifications using nanopore sequencing for mRNA and rRNA has been rapidly developing (Non-Patent Literature 1 to 4). Since RNA modifications in mRNA and rRNA are not frequent, RNA modification analysis using existing base callers is possible. Regarding tRNA, analyses using methods similar to those for mRNA and rRNA have also been reported (Non-Patent Literature 5 and 6).

## Citation List

## Non-Patent Literature

Non-Patent Literature 1: Liu, H. et al. Accurate detection of m6A RNA modifications in native RNA sequences. Nature communications 10, 4079, doi:10.1038/s41467-019-11713-9 (2019).

Non-Patent Literature 2: Stoiber, M. et al. De novo Identification of DNA Modifications Enabled by Genome-Guided Nanopore Signal Processing. bioRxiv, 094672, doi:10.1101/094672 (2017).

Non-Patent Literature 3: Leger, A. et al. RNA modifications detection by comparative Nanopore direct RNA sequencing. Nature Communications 12, 7198, doi:10.1038/s41467-021-27393-3 (2021).

Non-Patent Literature 4: Smith, A. M. et al. Reading canonical and modified nucleobases in 16S ribosomal RNA using nanopore native RNA sequencing. PLoS One 14, e0216709, doi:10.1371/journal.pone.0216709 (2019).

Non-Patent Literature 5: Thomas, N. K. et al. Direct Nanopore Sequencing of Individual Full Length tRNA Strands. ACS Nano 15, 16642-16653, doi:10.1021/acsnano.1c06488 (2021).

Non-Patent Literature 6: Lucas, M. C. et al. Quantitative analysis of tRNA abundance and modifications by nanopore RNA sequencing. Nat Biotechnol, doi:10.1038/s41587-023-01743-6 (2023).

## Summary of Invention

### Technical Problem

However, although detection of some tRNA modifications has been successful, a method for analyzing tRNA modifications at a single-molecule level has not yet reached a practical stage. One reason for this is that diverse modifications exist at high density in tRNA. Since the data analysis methods described in the above citation list do not have sufficient analysis accuracy when diverse modifications exist at high density, there is a demand for development of a new data analysis method entirely different from the conventional ones.

Accordingly, an object of the present invention is to provide a novel method and the like for analyzing a modification state of constituent units in a molecule in which a plurality of constituent units of one or more types are bonded together.

### Solution to Problem

An analysis method according to the present embodiment is a method for analyzing a modification state of a constituent unit in a molecule in which a plurality of constituent units of one or more types are bonded together, the method including: acquiring series data of signal values that vary in accordance with a type and a modification state of each constituent unit, the series data being obtained by scanning the molecule for each of a plurality of analyte molecules; aligning each of the plurality of series data, acquired from the plurality of analyte molecules, with reference data, thereby aligning the plurality of series data with each other; and analyzing a modification state of at least a part of the plurality of analyte molecules, based on the aligned plurality of series data.

In this analysis method, since the series data of signal values, which vary in accordance with a type and a modification state of each constituent unit, the series data being obtained by scanning the molecule, are aligned to compare the series data with each other, even when analyzing a molecule in which diverse modifications exist at high density, the analysis accuracy does not decrease as in conventional analysis methods, and the modification state can be analyzed with high accuracy. The plurality of analyte molecules may include one or more types of molecules. The plurality of analyte molecules may include a plurality of molecules of one type, and may include two or more types, each type having a plurality of molecules.

In the above method, it is preferable to further include generating the reference data by mixing a plurality of series data including the acquired series data. In this aspect, since the reference data used for aligning the plurality of series data is obtained by mixing a plurality of series data actually obtained by measurement, it is possible to create reference data having higher alignment accuracy with respect to the plurality of series data. In this aspect, it is not necessary to use all of the acquired plurality of series data for the mixing, and it is sufficient to mix at least some of the acquired series data (for example, one-third or more, one-half or more, or two-thirds or more of the total number).

In the above aspect, it is preferable that the plurality of series data to be mixed include two or more types of series data acquired or estimated from two or more types of molecules that have the same constituent unit sequence as that of at least one of the analyte molecules and that have different modification states of the constituent units from each other. In this aspect, since the reference data is obtained by mixing series data based on two or more molecules having different modification states, it is possible to create reference data having higher alignment accuracy with respect to the plurality of series data.

In the above method, the analyzing of the modification states of the plurality of analyte molecules may include calculating a distance between a distribution of signal values at a predetermined point in the plurality of series data relating to a first molecule in which the modification state of the constituent units is a first modification state, and a distribution of signal values at a point of the plurality of series data relating to a second molecule in which the modification state of the constituent units is a second modification state, with the point being associated with the predetermined point, by using the plurality of series data acquired or estimated from the first molecule and aligned, and the plurality of series data acquired or estimated from the second molecule and aligned, and estimating a portion having a different modification state between the first molecule and the second molecule, based on the calculated distance. In this aspect, it is possible to find the portion having a different modification state between the two types of series data.

In the above method, the analyzing of the modification states of the plurality of analyte molecules may include estimating a modification state of a first constituent unit in a third molecule, in which the modification state of the first constituent unit is unknown, by using a machine learning model trained with training data, the training data including the series data acquired from a first molecule in which the modification state of the first constituent unit existing at a first position in a constituent unit sequence of the analyte molecules is a first modification state and the series data acquired from a second molecule in which the modification state of the first constituent unit is a second modification state. In this aspect, it is possible to estimate the modification state of a constituent unit at a specific position in a molecule in which the modification state of the constituent unit is unknown.

In the above method, the analyzing of the modification states of the plurality of analyte molecules may include classifying the aligned plurality of series data into two or more types of clusters by using an unsupervised machine learning model. In this aspect, it is possible to classify molecules having unknown modification states into two or more types of clusters.

In the above aspect, the classifying into clusters may include calculating a similarity between the series data for each of the aligned plurality of series data, and classifying the aligned plurality of series data into two or more types of clusters based on the similarity. In this aspect, it is possible to perform clustering with less computation than in a case where the series data are used for clustering as they are.

In the above method, the molecules may be nucleic acids. In addition, it is preferable that the plurality of analyte molecules include at least two types of: nucleic acids derived from a wild-type strain; nucleic acids having no modification; and nucleic acids derived from a mutant strain whose modification state of a specific base has been altered from that of the wild-type strain. In addition, it is preferable that the plurality of analyte molecules include all of nucleic acids derived from a wild-type strain, nucleic acids having no modification, and nucleic acids derived from a mutant strain whose modification state of a specific base has been altered from that of the wild-type strain. In this aspect, it is possible to specify modified portions in nucleic acids derived from a wild-type strain on the series data.

In the above aspect, it is preferable that the plurality of analyte molecules include at least the nucleic acids derived from a wild-type strain, and that a proportion of the number of bases having modifications relative to a total number of bases constituting the nucleic acids derived from a wild-type strain be 4% or more. In this aspect, it is possible to effectively exhibit the effect of the above method in which the analysis accuracy is high when diverse modifications exist at high density.

In the above aspect, calculating redundancy of the series data relative to the reference data, when aligning each of the plurality of series data with the reference data, may be further included, and the modification state may be analyzed based on the calculated redundancy. In this aspect, the speed of scanning the molecule is expressed by the redundancy of the series data. Therefore, in this aspect, it is possible to analyze the modification states of the constituent units of the molecule based on a change in the scanning speed of the molecule.

A diagnostic support method according to the present embodiment is a method for supporting diagnosis of a disease that causes a disorder in a modification state of a constituent unit in a molecule in which a plurality of constituent units of one or more types are bonded together, the method including: analyzing a specimen containing the molecule and derived from a patient and a specimen containing the molecule and derived from a healthy subject, by the above analysis method; and analyzing a difference in the modification state of the constituent units in the molecule between the specimen derived from the patient and the specimen derived from the healthy subject. According to this diagnostic support method, the diagnosis of the above disease can be facilitated.

In the present specification and the like, the term "system" means a system composed of a plurality of devices and the like for providing a user with a specific function. For example, it may be configured by a server device, a cloud computing form, an Application Service Provider (ASP), a client-server model, or the like, but is not limited thereto.

In addition, the term "unit" is not limited to a physical configuration, and also includes a case where the function of the configuration is realized by software. In addition, the function of one configuration may be realized by two or more physical configurations, or the functions of two or more configurations may be realized by one physical configuration.

**Advantageous Effect of Invention**

According to the present invention, it is possible to provide a novel method and the like for analyzing a modification state of constituent units in a molecule in which a plurality of constituent units of one or more types are bonded together.

**Brief Description of Drawings**

[Figure 1] Figure 1 is a schematic diagram of an analysis system according to the present embodiment.

[Figure 2] Figure 2 is a functional block diagram of an analysis device according to the present embodiment.

[Figure 3] Figure 3 is a diagram showing a physical configuration of the analysis device according to the present embodiment.

[Figure 4] Figure 4 is a flowchart of processing in the analysis device according to the present embodiment.

[Figure 5] Figure 5 is an example of analysis results in an analysis method according to the present embodiment.

[Figure 6] Figure 6 is an example of the analysis method according to the present embodiment.

[Figure 7] Figure 7 shows the design of an adapter when measuring tRNA in an example using a nanopore sequencer. Figure 7(A) shows an overview of the design of the adapter. Figure 7(B) shows the design of a 1st adapter.

[Figure 8] Figure 8 shows an example in which modifications of *Escherichia coli* tRNA$^{Asn}$ were detected by the analysis method according to the present embodiment. Figure 8(A) shows modifications of *Escherichia coli* tRNA$^{Asn}$ and deletion strains corresponding to each modification. Figure 8(B) shows distances between distributions of signal values in tRNA$^{Asn}$ derived from a wild-type strain, and those in tRNA$^{Asn}$ derived from in vitro transcript and derived from each deletion strain.

[Figure 9] Figure 9 is an example of a machine learning model created for the modification of *Escherichia coli* tRNA$^{Gly3}$ using the analysis method according to the present embodiment.

[Figure 10] Figure 10 is an example of clustering by unsupervised machine learning regarding modifications of *Escherichia coli* tRNA$^{Pro3}$ by the analysis method according to the present embodiment.

[Figure 11] Figure 11 is an example of a method for calculating redundancy of series data with respect to reference data in the analysis method according to the present embodiment.

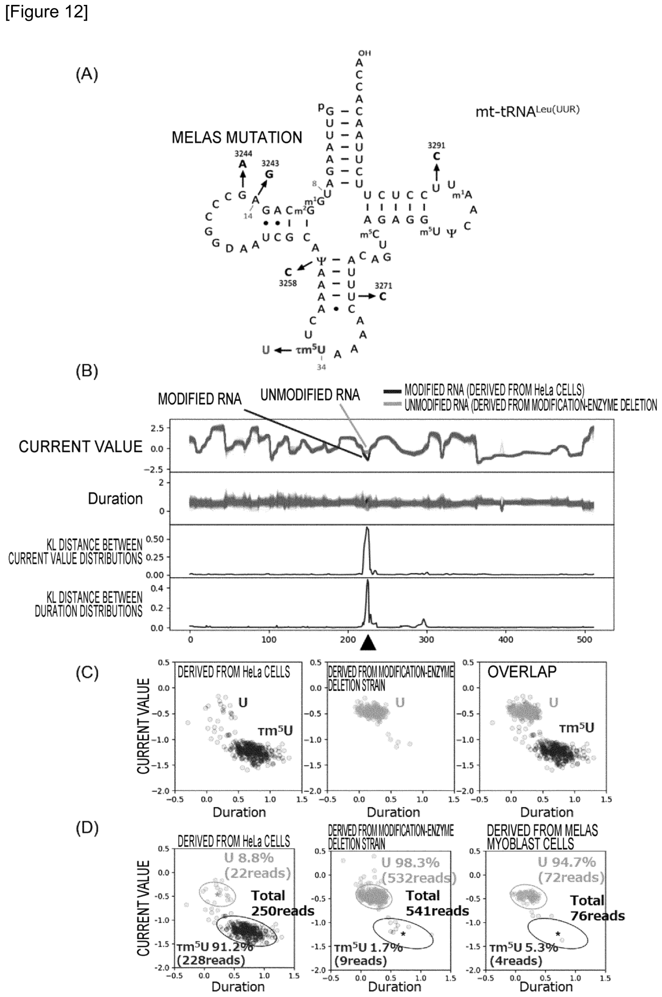

[Figure 12] Figure 12 shows an example in which modifications of human mt-tRNA$^{Leu(UUR)}$ were detected by the analysis method according to the embodiment. Figure 12(A) shows modifications of human mt-tRNA$^{Leu(UUR)}$. Figure 12(B) shows current values, Duration, and Kullback-Leibler (KL) distances between distributions thereof in tRNAs derived from HeLa cells and in tRNAs derived from modification-enzyme deletion strains. Figure 12(C) shows distributions of current values and Duration at a point indicated by a triangle in Figure 12(B). Figure 12(D) shows distributions of U and $\tau m^5 U$ and estimated abundance ratios thereof in series data derived from HeLa cells, series data derived from modification-enzyme deletion strains, and series data derived from myoblasts of a MELAS patient.

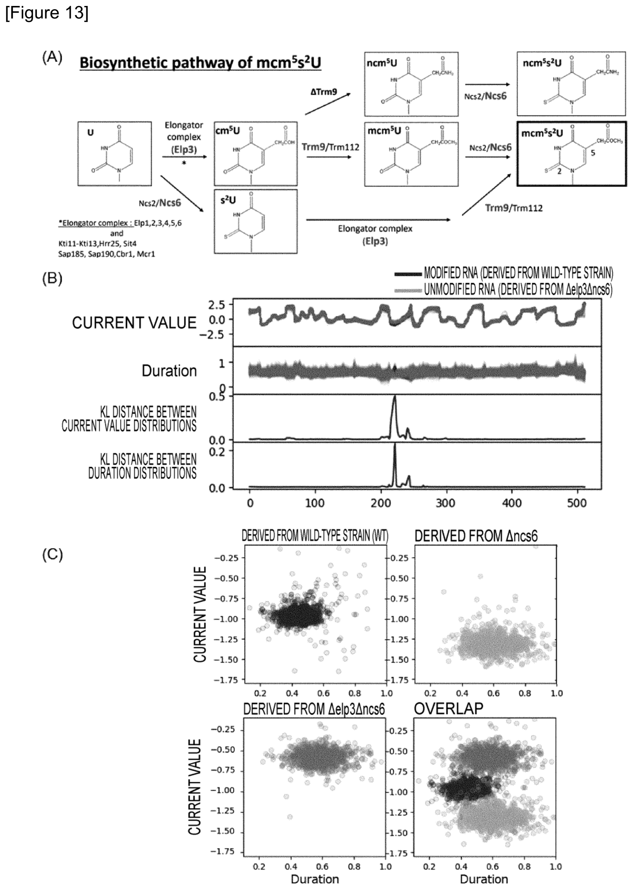

[Figure 13] Figure 13 shows an example in which modifications of yeast tRNA$^{Gln1a}$ were detected by the analysis method according to the embodiment. Figure 13(A) shows a biosynthetic pathway of $mcm^5 s^2 U$. Figure 13(B) shows current values, Duration, and Kullback-Leibler (KL) distances between distributions thereof in tRNAs derived from a wild-type strain and in tRNAs derived from a modification-enzyme deletion strain ($\Delta$elp3$\Delta$ncs6). Figure 13(C) shows distributions of current values and Duration at a peak position in Figure 13(B), in series data derived from a wild-type strain and in data derived from modification-enzyme deletion strains ($\Delta$elp3$\Delta$ncs6 and $\Delta$ncs6).

[Figure 14] Figure 14 shows an example in which modifications of yeast tRNA$^{Gln1a}$ were detected by the analysis method according to the embodiment. Distributions of each modification state and estimated abundance ratios in the wild-type strain and the modification-enzyme deletion strains ($\Delta$elp3$\Delta$ncs6 and $\Delta$ncs6) are shown.

**Description of Embodiments**

Hereinafter, an embodiment for implementing the present invention (hereinafter, referred to as "the present embodiment") will be described in detail with reference to the drawings. However, the present invention is not limited thereto, and various modifications may be made within the scope without departing from the gist thereof. In the following description of the drawings, the same or similar parts are denoted by the same or similar reference numerals.

[Analysis system]

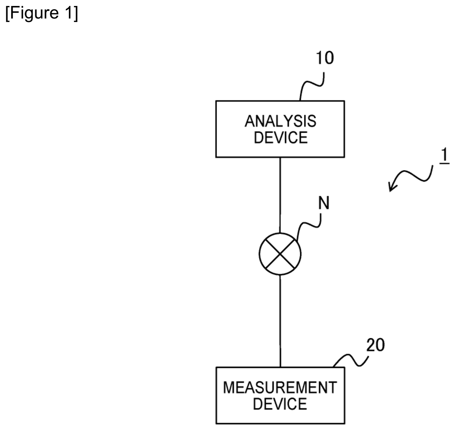

Figure 1 is a diagram showing an overview of the configuration of an analysis system 1 according to the present embodiment. The analysis system 1 includes a measurement device 20 and an analysis device 10 that analyzes measurement results acquired by the measurement device 20. In the analysis system 1, the measurement device 20 measures analyte molecules, and the analysis device 10 analyzes the measurement results, thereby analyzing modification states of the analyte molecules.
In the analysis system 1, the analysis device 10 is connected to the measurement device 20 via a communication network N. Here, the communication network N may be a wired or wireless communication network. The analysis device 10 does not necessarily have to be a device independent from the measurement device 20, and may instead be configured integrally with the measurement device 20. In such a case, the measurement device may also function as the analysis device 10 by executing an analysis program installed in the measurement device.

The analysis system 1 according to the present embodiment analyzes modification states of constituent units in a molecule in which a plurality of constituent units of one or more types are bonded together (hereinafter, simply referred to as "molecule"). The molecule analyzed by the analysis system 1 is preferably a biomacromolecule. Examples of biomacromolecules include peptides, proteins, nucleic acids, polysaccharides, and combinations thereof (for example, lipoproteins, glycoproteins, nucleoproteins, and glycans). Here, constituent units of peptides and proteins are amino acids, constituent units of nucleic acids are nucleotides, and constituent units of polysaccharides are monosaccharides. It is preferable that the biomolecules be capable of being single-stranded.

In the present specification, "modification" of a molecule or a constituent unit thereof means that the molecule or the constituent unit thereof is changed from its normal state, for example, from a natural or wild-type state. Modifications include, for example, addition of an additional group not normally present, deletion of a group normally present, or substitution of an atom or atomic group normally present with another atom or atomic group. For example, modification of an amino acid may be modification of a side chain of the amino acid. Examples of such amino acid modifications include post-translational modifications such as phosphorylation, methylation, acetylation, and glycosylation. Modification of a nucleotide may be modification of a sugar moiety of a nucleoside and/or a base moiety of a nucleoside. Modification of a monosaccharide may be modification of a hydrocarbon chain. Modifications of nucleotides and the like include post-transcriptional modifications in RNA molecules, and epigenetic modifications in DNA molecules or histone proteins covering DNA molecules. Examples of modifications of glycans include phosphorylation, methylation, acetylation, glycosylation, and sulfonation.

The molecules whose modification states are analyzed by the analysis system 1 may be any biomolecules capable of being single-stranded, but nucleic acids are preferable, and tRNAs are particularly preferable. In many cases, diverse modifications exist at high density in tRNAs, and the analysis by the analysis system 1 according to the present embodiment is particularly effective. The molecules whose modification states are analyzed by the analysis system 1 are preferably molecules in which, in a molecule derived from a wild-type strain, a proportion of the number of constituent units having modifications relative to the total number of constituent units constituting the molecule is 4% or more, 5% or more, 6% or more, 7% or more, 8% or more, 9% or more, or 10% or more.

Modifications in nucleic acids may be referred to in Boccaletto, P. et al. MODOMICS: a database of RNA modification pathways. 2021 update. Nucleic Acids Res 50, D231-D235, doi:10.1093/nar/gkab1083 (2022), or the like.

The analysis device 10 according to the present embodiment analyzes measurement results by a completely different method from conventional analysis devices, and analyzes modification states of analyte molecules.

For example, in conventional analysis devices that analyze modification states of nucleic acids, a base sequence is estimated by base calling from actually measured current signals, the estimated base sequence is aligned with a reference sequence based on similarity, and feature values of each base are obtained. Since modified bases exhibit current values different from unmodified bases, the accuracy of mapping to the reference sequence based on similarity decreases in modified base portions. When analyzing modification states of mRNAs and rRNAs, since the frequency of RNA modifications is low and most bases are unmodified, mapping accuracy of modified bases is consequently secured by correctly mapping portions other than modified bases. On the other hand, in cases such as tRNAs, in which many types and numbers of RNA modifications exist, and a plurality of modified bases are densely clustered within short regions, such existing strategies cannot be applied.

On the other hand, the analysis device 10 according to the present embodiment does not estimate constituent units from signal values as in conventional analysis devices, but instead aligns a plurality of actually measured series data and performs signal-based analysis. Therefore, even when diverse modifications exist at high density, high-accuracy alignment can be achieved, and as a result, high-accuracy analysis can be realized.

Hereinafter, the measurement device 20 and the analysis device 10 will be described in detail, and a processing flow in the analysis system 1 according to the present embodiment will be explained.

(Measurement device)

The measurement device 20 is a device that measures a molecule in which a plurality of constituent units of one or more types are bonded together, on a per-molecule basis, and obtains series data. In order to obtain series data, the measurement device 20 acquires signal values that vary according to the types and modification states of the constituent units while scanning a molecule. The series data acquired by the measurement device 20 are typically time-series data, but are not necessarily limited to time-series data.

The measurement device 20 measures a plurality of molecules to be analyzed. Therefore, when analyte molecules are measured by the measurement device 20, a plurality of series data are obtained. The measurement device 20 may measure a plurality of molecules of one type, or may measure a plurality of molecules of two or more types.

Examples of the measurement device 20 include a nanopore sequencer. A nanopore sequencer acquires signal values reflecting the types and modification states of the constituent units of a molecule to be measured by measuring current values when the molecule to be measured passes through a nanopore of a size that allows passage of a single molecule to be measured, the nanopore being formed of membrane proteins or the like. The series data acquired by the nanopore sequencer are time-series data. For example, when a molecule passes through the nanopore from one end toward the other, signal values corresponding to portions of the molecule (that is, portions composed of one or more constituent units) passing through the nanopore are obtained, and as the molecule portions move from one end toward the other, time-series data are output.

As the measurement device 20, a commercially available device may be used. For example, a nanopore sequencer from Oxford nanopore technologies (ONT) can be used.

(Analysis device)

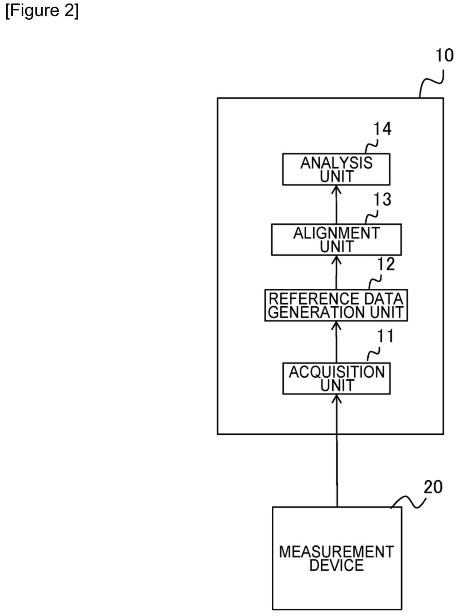

The analysis device 10 analyzes series data measured by the measurement device 20 and analyzes modification states of molecules measured. Figure 2 is a functional block diagram of the analysis device 10. The analysis device 10 includes an acquisition unit 11, a reference data generation unit 12, an alignment unit 13, and an analysis unit 14. The analysis device 10 may not have, for example, the reference data generation unit 12, or may have another functional configuration not shown in Figure 2.

The acquisition unit 11 acquires series data measured by the measurement device 20. The acquisition unit 11 may be implemented by a communication unit 10d described below. The acquisition unit 11 may acquire the series data measured by the measurement device 20 for each measurement, or may collectively acquire a plurality of series data obtained by a plurality of measurements.

The reference data generation unit 12 generates reference data used for alignment in the alignment unit 13. The reference data generation unit 12 generates reference data alignable with each of a plurality of series data measured by the measurement device 20. The reference data may, for example, include any one of the plurality of series data measured by the measurement device 20, and may include series data estimated from sequence information of a molecule measured by the measurement device 20. For example, the reference data may be any one of the plurality of series data measured by the measurement device 20, may be the series data estimated from sequence information of a molecule measured by the measurement device 20, or may be series data obtained by mixing two or more of the plurality of series data measured by the measurement device 20 and, optionally, the series data estimated from sequence information of a molecule measured by the measurement device 20. The reference data generation unit 12 may be implemented by a program stored in a RAM 10b or a ROM 10c described below and executed by a CPU 10a.

The alignment unit 13 aligns the plurality of series data acquired by the acquisition unit 11 based on the reference data generated by the reference data generation unit 12. In the present specification, the term "to align" series data means to make the overall length of the data uniform by associating data points between the data while maintaining the order of each data point, for series data where direct comparison is difficult due to a difference in the overall data length caused by a difference in the length or number of corresponding data points, even though a correspondence exists in the order of each data point. For example, in the case of time-series data, alignment means identifying corresponding data points between two series data and adjusting the time of each data point so that the times of corresponding data points match, thereby aligning the overall data length.

The alignment unit 13 may be implemented by an input unit 10e described below and the program stored in the RAM 10b or the ROM 10c described below and executed by the CPU 10a. The alignment unit 13 may perform alignment by, for example, a dynamic programming method, or by a dynamic time warping (DTW) method. The DTW is not particularly limited, but soft-DTW is preferable. In addition, series data obtained by the measurement device 20 may be interpreted as state-transition data according to a hidden Markov model, and alignment may be performed by a Viterbi algorithm.

The alignment unit 13 may calculate redundancy of series data relative to reference data when aligning a plurality of series data based on the reference data. Here, redundancy of series data relative to reference data means the length (the number of data points) of portions of series data associated with predetermined portions of reference data. For example, when two or more points of series data are associated with one point of reference data, compared with when one point of series data is associated with two or more points of reference data, the former can be said to have higher redundancy of series data than the latter.

The redundancy of series data relative to reference data, which is associated with predetermined portions of the reference data, is locally calculated for each portion of the reference data and the series data. A specific example of a method for calculating the redundancy of series data will be described below.

The analysis unit 14 analyzes modification states of molecules corresponding to at least some of the acquired series data, based on a plurality of series data aligned by the alignment unit 13. Since a plurality of series data acquired by the acquisition unit 11 are aligned by the alignment unit 13, the analysis unit 14 can analyze modification states with high accuracy. Specifically, the analysis unit 14 detects modifications based on changes in signal values and/or changes in redundancy using the plurality of aligned series data. Details of the analysis method in the analysis unit 14 will be described below. The analysis unit 14 may be implemented by the input unit 10e described below and the program stored in the RAM 10b or the ROM 10c described below and executed by the CPU 10a.

The analysis device 10 may output analysis results from the analysis unit 14 in an appropriate format. For example, the analysis device 10 may display analysis results on a display unit 10f described below, or may transmit analysis results to another terminal via the communication unit 10d. The analysis results may be in the form of three-dimensional or two-dimensional graphs, numerical data, or modification states of constituent units in molecules.

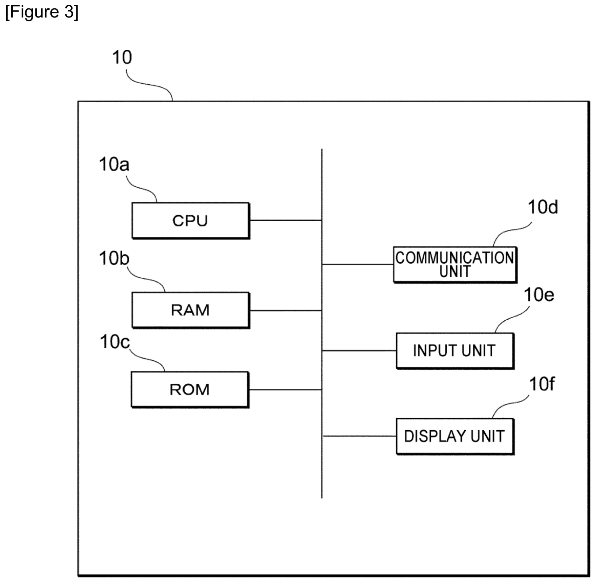

Figure 3 is a diagram showing a physical configuration of the analysis device 10. The analysis device 10 has a central processing unit (CPU) 10a which corresponds to a processor, a random access memory (RAM) 10b and a read-only memory (ROM) 10c which correspond to storage units, a communication unit 10d, an input unit 10e, and a display unit 10f. These components are connected to each other via a bus to enable data transmission and reception. In this example, the analysis device 10 is described as being configured by a single computer, but the analysis device 10 may be realized by combining a plurality of computers. In addition, the configuration shown in Figure 3 is an example, and the analysis device 10 may include other configurations or may not include some of these configurations.

The CPU 10a is a control unit that performs control related to execution of programs stored in the RAM 10b or the ROM 10c, and performs data computation and processing. The CPU 10a is a computation unit that executes a program (analysis program) for analyzing series data acquired by the measurement device 20. The CPU 10a receives various data from the input unit 10e and the communication unit 10d, displays results of data computation on the display unit 10f, or stores them in the RAM 10b or the ROM 10c.

The RAM 10b is a memory unit in which data can be rewritten, and may be configured to have, for example, a semiconductor memory element. The RAM 10b may store the analysis program executed by the CPU 10a. These are merely examples, and the RAM 10b may store other data as well.

The ROM 10c is a memory unit in which data can be read, and may be configured to have, for example, a semiconductor memory element. The ROM 10c may store, for example, data that is not rewritten.

The communication unit 10d is an interface for connecting the analysis device 10 to another device. The communication unit 10d may be connected to the communication network N such as the Internet.

The input unit 10e receives data input from a user, and may include, for example, a keyboard and a touch panel.

The display unit 10f visually displays computation results by the CPU 10a, and may be configured to have, for example, a liquid crystal display (LCD). The display unit 10f may display analysis results from the analysis unit 14.

The analysis program may be stored and provided in a computer-readable storage medium such as the RAM 10b or the ROM 10c, or may be provided via a communication network connected by the communication unit 10d. In the analysis device 10, the CPU 10a executes the analysis program, thereby realizing operations of the acquisition unit 11, the reference data generation unit 12, the alignment unit 13, and the analysis unit 14 described with reference to Figure 2. These physical configurations are merely examples and do not necessarily have to be independent. For example, the analysis device 10 may include a large-scale integration (LSI) in which the CPU 10a and the RAM 10b and/or the ROM 10c are integrated.

(Processing flow)

<Overview>

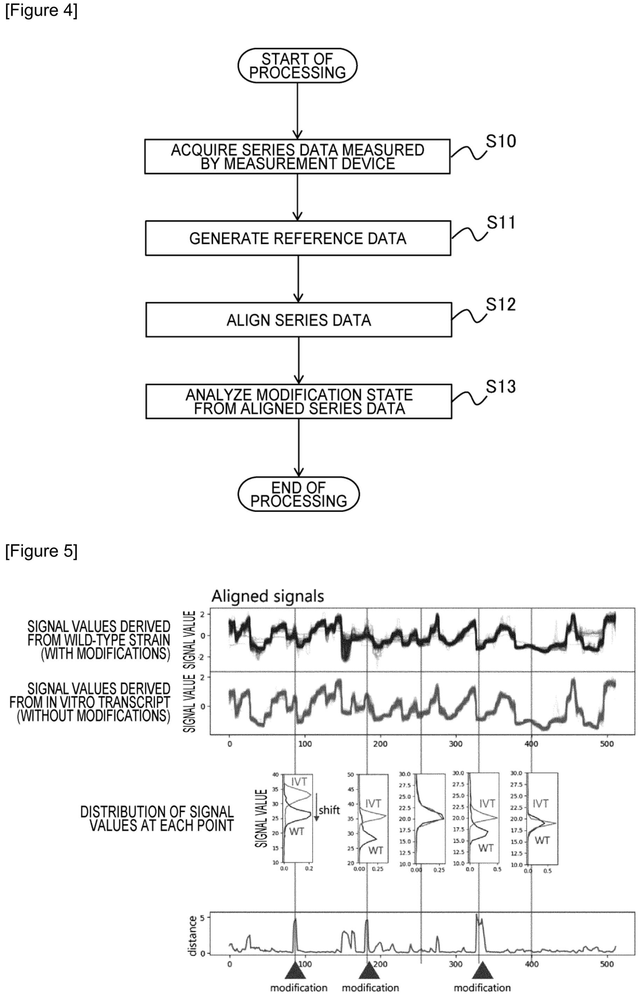

Figure 4 is a flowchart of processing in the analysis device 10 according to the present embodiment. First, the acquisition unit 11 acquires a plurality of series data from the measurement device 20 (S10). Before Step S10, the measurement device 20 measures one type or two or more type of analyte molecules and measures series data. When there are two or more types of analyte molecules, it is assumed that the two or more types of analyte molecules have the same sequence of constituent units but differ from each other in modification states of the constituent units. For example, when the analyte molecules are nucleic acids, the measurement device 20 measures two or more types of nucleic acids having the same base sequence and different modification states.

When the measurement device 20 measures two or more types of analyte molecules, the two or more types of analyte molecules may be measured one by one to acquire a plurality of series data labeled for each type, or two or more types of analyte molecules may be collectively measured to acquire a plurality of unlabeled series data.

Next, the reference data generation unit 12 generates reference data for aligning the series data acquired in Step S10 (S11). The reference data to be generated is not particularly limited as long as it can be aligned with each of the acquired series data.

In Step S11, for example, the reference data generation unit 12 may generate reference data by any of the following methods.

(1) A method of selecting any one of the series data acquired in Step S10 as reference data.
In this method, the reference data may be randomly selected from among the series data acquired in Step S10, or may be selected based on the length of the series data. For example, the series data located at the center when the acquired series data is arranged in order of length of the series data may be selected as reference data.

(2) A method of generating reference data based on the sequence of constituent units of the analyte molecule, when the sequence is known.

This method is a method of generating virtual series data predicted from the sequence of constituent units of the analyte molecule and using it as reference data. For example, when the analyte molecule is a nucleic acid, software can be used to virtually predict current values in a nanopore sequencer based on the base sequence. Reference data can be generated by this method only when the correspondence between the sequence data and the signal values to be measured is known. For example, when the analyte molecule is a nucleic acid, series data in the case where the nucleic acid is unmodified can be predicted by a known method.

(3) A method of generating reference data by mixing at least some of the series data acquired in Step S10.

In this method, a plurality of series data obtained from one type of analyte molecule may be mixed, or a plurality of series data obtained from two or more types of analyte molecules may be mixed. For mixing the plurality of series data, an algorithm for obtaining the average of the plurality of series data may be used. Examples of such an algorithm include DTW barycenter averaging (DBA). In addition, when the series data obtained by the measurement device 20 are interpreted as state-transition data according to a hidden Markov model, parameters of the hidden Markov model may be estimated by a Baum-Welch algorithm.

In this method, it is preferable to mix two or more types of series data acquired from two or more types of molecules having the same sequence of constituent units but differing from each other in modification states of the constituent units. The reference data thus obtained tends to be aligned with high accuracy with respect to a plurality of series data because it has average characteristics of the two or more types of analyte molecules. In addition, in this method, it is more preferable to mix two or more types of series data acquired from at least two types of molecules among molecules derived from a wild-type strain, unmodified molecules, and molecules derived from a mutant strain whose modification states of specific bases have been altered from those of the wild-type strain. It is still more preferable to mix two types of series data acquired from molecules derived from a wild-type strain and unmodified molecules. It is not necessary to use all of the series data acquired in Step S10 for the mixing, and it is sufficient to mix at least some of the acquired series data (for example, one-third or more, one-half or more, or two-thirds or more of the total number).

(4) A method of generating reference data by virtually generating series data based on a known sequence of constituent units of an analyte molecule in the same manner as in (2) above, and further mixing the virtual series data and at least some of the series data acquired in Step S10 in the same manner as in (3) above.

In this method, the molecule for which the virtual series data is generated may be an unmodified molecule. The series data mixed with the virtual series data may be one or more types of series data acquired in Step S10.

Among the above methods (1) to (4), from the perspective of enabling high-accuracy alignment in Step S12 described below, the above methods (3) and (4) are preferable, the above method (3) is more preferable, and a method in (3) above in which two or more types of series data acquired from two or more types of molecules having the same sequence of constituent units but differing from each other in modification states of the constituent units is particularly preferable.

Next, the alignment unit 13 aligns the plurality of series data acquired in Step S10 based on the reference data generated in Step S11 (S12). In Step S12, the plurality of series data are aligned based on the reference data. More specifically, by aligning each of the plurality of series data with the reference data, the lengths of the plurality of series data are made to correspond to the length of the reference data, thereby facilitating comparison among the series data. The alignment can be performed as described above.

In Step S12, in addition to the plurality of series data acquired in Step S10, the virtual series data generated in Step S11 or series data stored in the RAM 10b or the ROM 10c may also be aligned with the reference data.

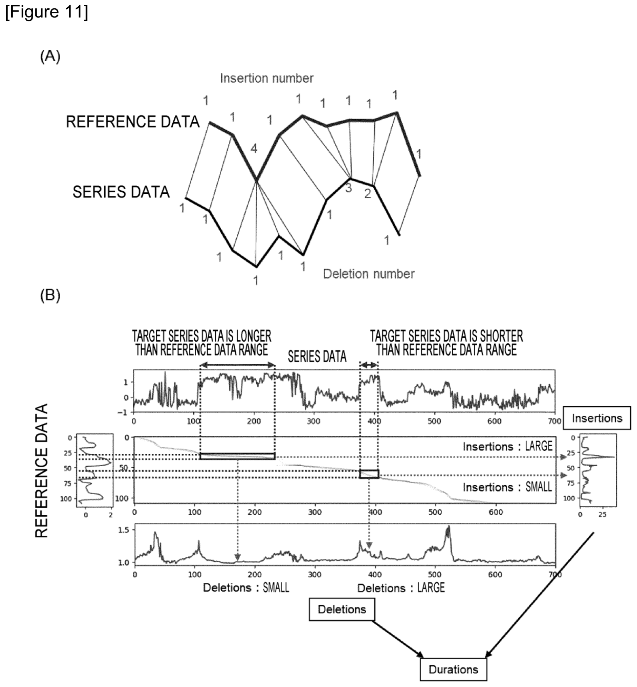

In addition, in Step S12, the redundancy of the series data relative to the reference data when aligning the plurality of series data based on the reference data may be calculated. The calculation of redundancy will be described with reference to Figure 11.

Figure 11(A) is a conceptual diagram illustrating alignment between the reference data and the series data. In Figure 11(A), each point of the reference data is associated with each point of the series data, and such associations are represented by line segments connecting the respective points. In the data shown in Figure 11(A), conceptually depicted are, for example, a portion where four points of the series data are associated with one point of the reference data, and a portion where one point of the series data is associated with three points of the reference data. In addition, a value indicating how many points in the series data are associated with one point of the reference data is indicated as the "Insertion number," and a value indicating how many points in the reference data are associated with one point of the series data is indicated as the "Deletion number."

By obtaining such Insertion numbers and Deletion numbers, it is possible to calculate redundancy of series data relative to reference data. The larger the Deletion number corresponding to each point in the series data, the lower the redundancy of the series data relative to the reference data (conversely, the reference data is redundant relative to the series data). The larger the Insertion number corresponding to a point in the reference data associated with each point in the series data, the higher the redundancy of the series data relative to the reference data.

Next, with reference to Figure 11(B), an example will be described in which redundancy of series data relative to reference data is calculated using a correspondence matrix between the reference data and the series data to be aligned. The correspondence matrix can be obtained by, for example, a soft DTW algorithm using the reference data and the series data.

In Figure 11(B), a correspondence matrix is shown in which the strength of correspondence between each point in the reference data and each point in the series data to be aligned is probabilistically represented with values ranging from 0 to 1. The matrix can be expressed as a matrix $P = (p_{ij})$ (i: integer from 1 to n, which is the number of data points in the series data; j: integer from 1 to m, which is the number of data points in the reference data). For example, when the first point in the reference data is associated with the first and second points in the series data, the first row of the matrix P has entries of 1 in the first and second columns, and the other entries are 0. In the correspondence matrix shown in Figure 11(B), areas with probabilities close to 1 are shown in darker color.

Using such a matrix P, the Deletion number at the i-th point in the series data can be expressed by the following Equation (I), and the Insertion number at the j-th point in the reference data can be expressed by the following Equation (II).

[Equation 1]

$$Deletion_i = \sum_{k=1}^{m} p_{ik} \qquad (I)$$

$$Insertion_j = \sum_{k=1}^{n} p_{kj} \qquad (II)$$

In addition, by calculating the weighted average of the Deletion number of the series data using the following Equation (III) and matching the length of the Deletion number with the length of the reference data, the Duration reflecting both the Deletion number and the Insertion number can be calculated using the following Equation (IV). Duration serves as an index indicating the degree of local redundancy of the point in the series data associated with the j-th point in the reference data. When the Duration is 0, it means that the series data and the reference data are associated on a one-to-one basis. When the Duration is less than 0, it means that the reference data is redundant relative to the series data. When the Duration is greater than 0, it means that the series data is redundant relative to the reference data.

[Equation 2]

$$aligned\ Deletion_j = \frac{\sum_{i=1}^{n} p_{ij} \times Deletion_i}{\sum_{i=1}^{n} p_{ij}} \qquad (III)$$

$$Duration_j = \log_{10} \frac{Insertion_j}{aligned\ Deletion_j} \qquad (IV)$$

By using the above Equations (I) to (IV), as shown in Figure 11(B), local redundancy of the series data and the reference data can be displayed as a graph corresponding to the horizontal axes of the series data and the reference data.

Returning to Figure 4, next to Step S12, the analysis unit 14 analyzes at least a part of the modification state of the analyte molecule based on the plurality of series data aligned in Step S12 (S13). Since the series data have been aligned in Step S12, in this Step S13, it becomes easier to statistically detect changes in signal values due to the type of the analyte molecule and/or changes in redundancy of the series data relative to the reference data. Examples of this Step S13 will be described below.

Analyzing at least a part of the modification state of the analyte molecule based on the aligned plurality of series data includes, as described in detail below, analyzing the modification state by comparing the magnitudes and distributions of signal values of the plurality of series data, analyzing the modification state by comparing the degree and distribution of redundancy of the plurality of series data relative to the reference data, or analyzing the modification state using a machine learning model that takes the aligned plurality of series data as input values.

<Modification state analysis step>

(First aspect)

Step S13 may include, for example, calculating a distance between a distribution of signal values at a predetermined point in a plurality of series data relating to a first molecule in which the modification state of the constituent units is a first modification state, and a distribution of signal values at a point of a plurality of series data relating to a second molecule in which the modification state of the constituent units is a second modification state, with the point being associated with the predetermined point, by using the plurality of series data acquired from the first molecule and aligned, and the plurality of series data acquired from the second molecule and aligned, and estimating a portion having a different modification state between the first molecule and the second molecule, based on the calculated distance. Here, the series data of the first molecule and/or the series data of the second molecule may be virtual series data generated by a method similar to (2) in Step S11 above, but preferably, at least one is the series data acquired in Step S10, and more preferably, both are the series data acquired in Step S10.

Figure 5 is a diagram illustrating an example of analysis in this aspect, in a case where the first molecule is a molecule derived from a wild-type strain (a natural molecule) and the second molecule is a molecule derived from an in vitro transcript (a molecule synthesized in a test tube). In Figure 5, a plurality of series data acquired from the molecule derived from a wild-type strain and aligned, and a plurality of series data acquired from the molecule derived from an in vitro transcript and aligned are shown in the upper section. In the middle section, at several points of the series data, a distribution of signal values in the series data of the molecule derived from a wild-type strain (WT) and a distribution of signal values in the series data of the molecule derived from an in vitro transcript (IVT) are shown. In the graph in the middle section, the vertical axis represents the signal value, and the horizontal axis represents the frequency. The difference in the distribution of signal values between WT and IVT indicates that the modification states of the molecule derived from a wild-type strain and the molecule derived from an in vitro transcript are different at that portion. In the lower section, distances between the distribution of signal values in the series data of the molecule derived from a wild-type strain and the distribution of signal values in the series data of the molecule derived from an in vitro transcript are shown for all points of the series data. As emphasized by triangles in the lower part of the distance graph, portions where the calculated distance exceeds a predetermined threshold can be estimated as modification sites of the molecule derived from a wild-type strain.

In this aspect, as described above, at a predetermined point in a plurality of aligned series data relating to two or more types of molecules, the distance between the distributions of signal values in the series data among the various types of molecules is calculated. In the method of the present embodiment, since the plurality of series data are aligned, it is easy to compare corresponding points in the series data. For example, referring to the upper graph in Figure 5, by comparing points having the same horizontal axis value, the distance between the distributions of signal values in the series data among the various types of molecules can be easily calculated. In this aspect, the distances between the distributions of signal values in the plurality of series data relating to two or more types of molecules may be calculated for all points of the series data (for all values on the horizontal axis in the upper section of Figure 5).

The distance between the distributions of signal values refers to a value that quantitatively represents a difference between two or more distributions, and can be calculated by a known method. Examples of such methods include methods for quantifying a difference between two probability distributions. Specific examples thereof include Kullback-Leibler (KL) distance and Jensen-Shannon (JS) distance.

As described above, by calculating the distance between the distributions of signal values of two or more types of molecules, it is possible to estimate locations to be noted as portions having different modification states. Therefore, hereinafter, data obtained by calculating the distance between the distributions of signal values of two or more types of molecules and associating the calculated distance with each point of the series data may be referred to as an "attention map."

(Second aspect)

Step S13 may include, for example, estimating a modification state of a first constituent unit in a third molecule, in which the modification state of the first constituent unit is unknown, by using a machine learning model trained with training data, the training data including the series data acquired from a first molecule in which the modification state of the first constituent unit existing at a first position in a constituent unit sequence is a first modification state and the series data acquired from a second molecule in which the modification state of the first constituent unit is a second modification state.

In this aspect, for example, the first molecule may be a molecule derived from a wild-type strain, and the second molecule may be a molecule derived from an in vitro transcript or a molecule derived from a deletion strain in which an enzyme involved in modification at the above first position in the wild-type strain has been deleted.

The training data for the machine learning model in this aspect are series data labeled as either the first or the second molecule. The machine learning model may perform, as in the above first aspect, weighting by using an attention map created from the molecule derived from a wild-type strain and either the molecule derived from an in vitro transcript or the molecule derived from a mutant strain whose modification state of a constituent unit at a specific position has been altered from that of the wild-type strain. In such a case, portions with larger distances in the attention map may be weighted more heavily.

(Third aspect)

Step S13 may include, for example, classifying the aligned plurality of series data into two or more types of clusters by using an unsupervised machine learning model. In this aspect, clustering may be performed directly on the aligned plurality of series data, or the similarity between the aligned plurality of series data may be calculated and classifying may be performed based on the similarity.

The similarity between two series data may be calculated by a known method; for example, a distance (the absolute value of the difference in signal values) at each point in the aligned series data may be calculated, and the sum of the distances at all points may be used as an index of similarity. When clustering is performed based on similarity, for example, taking a case of clustering N series data as an example, the similarity between each molecule and the N series data including itself may be quantified (for example, by calculating distances as described above), and N vectors each having N components with the similarity as a component may be generated, and the vectors may be clustered. Note that N is not particularly limited as long as it is 2 or more.

The method for clustering series data or vectors having similarity as a component is not particularly limited, and for example, methods such as hierarchical clustering, k-means, and density-based spatial clustering of applications with noise (DBSCAN) may be used.

In addition, for example, when the origin of the series data is known at the time of acquisition of the series data, the machine learning model may perform, as in the above first aspect, weighting by using an attention map created from a molecule derived from a first strain and a molecule derived from a second strain. The first molecule may be a molecule derived from a wild-type strain, and the second molecule may be a molecule derived from an in vitro transcript or a molecule derived from a deletion strain in which an enzyme involved in modification at the above first position in the wild-type strain has been deleted.

(Fourth aspect)

Step S13 may, for example, analyze the modification state based on the redundancy of the series data calculated in Step S12. For example, taking a case where the analyte molecule is RNA as an example, when nanopore sequencing measurement is used for RNA, the speed at which the RNA passes through the nanopore changes depending on whether or not the RNA is modified. One of the factors is that the speed at which an RNA double strand is unwound into a single strand by a helicase when RNA passes through the nanopore varies depending on the presence or absence of RNA modifications. For example, it has been reported that when RNA having a 2'-O-methyl modification passes through the helicase, the transit time of the base (9-11 bases downstream) being read inside the nanopore increases (Stephenson W, Razaghi R, Busan S, Weeks KM, Timp W, Smibert P (2022) Direct detection of RNA modifications and structure using single-molecule nanopore sequencing. Cell Genom 2).

In this manner, since the speed at which the analyte molecule is scanned can change depending on the modification state of the analyte molecule, the series data obtained by scanning the analyte molecule contain information regarding the modification state of the analyte molecule not only in the magnitude of signal values but also in the length of the series data, in other words, in the redundancy relative to the reference data. Therefore, in this aspect, analysis of the modification state is performed based on the redundancy of the series data calculated in Step S12.

In this aspect, for example, the analysis may be performed in the same manner as in the above first aspect, except that the distance between the redundancies of the series data is calculated instead of calculating the distance between the distributions of signal values in the series data. That is, this aspect may include, for example: calculating the redundancy of the series data relative to the reference data as in Step S12 for each of a plurality of series data acquired from a first molecule in which the modification state of the constituent units is a first modification state and aligned, and a plurality of series data acquired from a second molecule in which the modification state of the constituent units is a second modification state and aligned; calculating a distance between a distribution of the redundancy at a predetermined point in the plurality of series data relating to the first molecule and a distribution of the redundancy at a point in the plurality of series data relating to the second molecule, the point being associated with the predetermined point; and estimating a portion having a different modification state between the first molecule and the second molecule based on the calculated distance. Here, the series data of the first molecule and/or the series data of the second molecule may be virtual series data generated by a method similar to (2) in Step S11 above, but preferably, at least one is the series data acquired in Step S10, and more preferably, both are the series data acquired in Step S10.

Alternatively, in this aspect, for example, the analysis of the modification state may be performed by combining the analysis in the above first aspect and the analysis based on the redundancy of the series data. For example, for a first molecule in which the modification state of the constituent units is a first modification state and a second molecule in which the modification state of the constituent units is a second modification state, estimating the difference in the modification state between the first molecule and the second molecule based on the distribution of signal values at a first point and the distribution of redundancy at a second point in the plurality of series data relating to the first molecule, and the distribution of signal values at a point in the plurality of series data relating to the second molecule, the point being associated with the first point, and the distribution of redundancy at a point in the plurality of series data relating to the second molecule, the point being associated with the second point may be included. Here, the first point and the second point may be the same as or different from each other. For example, the first point may be a point at which the distance between the distributions of signal values in the first and second molecules is the greatest (the peak in the graph of the distance between distributions of signal values), and the second point may be a point at which the distance between the distributions of redundancy in the first and second molecules is the greatest (the peak in the graph of the distance between distributions of redundancy).

Specifically, at a predetermined point in the aligned series data, signal values may be plotted on a first axis (for example, the vertical axis) and redundancy may be plotted on a second axis (for example, the horizontal axis) to create a scatter diagram that reflects signal values and local redundancy. Based on this scatter diagram, a difference in modification state between the first molecule and the second molecule may be estimated. Further, the abundance ratio of the first molecule and the second molecule in a specimen may also be estimated from each plot in the scatter diagram. Examples of such a method include a method for clustering each plot in the scatter diagram. Specifically, for example, assuming that the current values and the Duration values are distributed according to a single bivariate normal distribution for each modification state, the modification state of each plot in the scatter diagram may be labeled using a bivariate Gaussian mixture model.

Alternatively, for example, for a first molecule in which the modification state of the constituent units is a first modification state and a second molecule in which the modification state of the constituent units is a second modification state, as described in the above first aspect and this aspect, respectively calculating the distance between the distribution of signal values and redundancy at a predetermined point in the plurality of series data relating to the first molecule, and the distribution of signal values and redundancy at a point in the plurality of series data relating to the second molecule, the point being associated with the predetermined point, and estimating a portion having a different modification state between the first molecule and the second molecule based on the two calculated distances may be included.

<Analysis example>

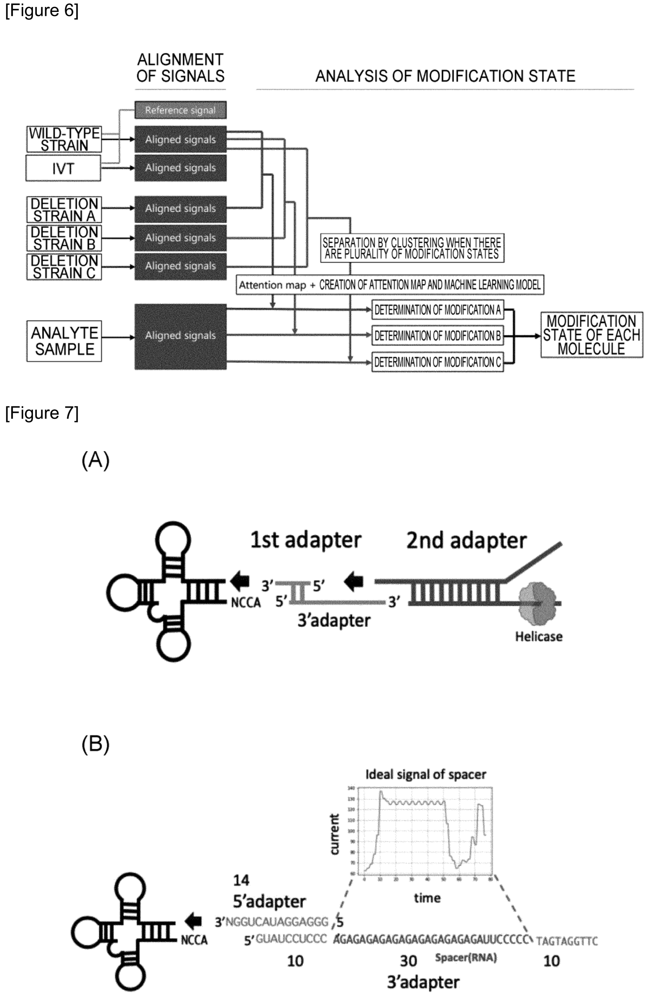

Figure 6 illustrates one example of analysis in the analysis system 1 according to the present embodiment. In the method shown in Figure 6, series data are acquired by the measurement device 20 for: a molecule derived from a wild-type strain, a molecule derived from an in vitro transcript (IVT), molecules derived from deletion strains (deletion strains A to C) in which the enzyme responsible for modification at a specific position in the wild-type strain has been deleted, and an analyte sample. Here, the modification state of the analyte sample is unknown, and the sequence of constituent units is the same for all molecules.

In the method shown in Figure 6, reference data are created by mixing the molecule derived from a wild-type strain and the molecule derived from an in vitro transcript (IVT), and all the above series data are aligned based on the reference data. By analyzing the molecule derived from a wild-type strain and each of the molecules derived from deletion strains using the method described in the modification state analysis step above, or the like, corresponding attention maps and a machine learning model are created. Using the created attention maps and machine learning model to analyze the analyte sample enables analysis of the modification state of the analyte sample on a per-molecule basis.

Although Figure 6 shows deletion strains A to C, the number of molecules derived from deletion strains compared with the molecule derived from a wild-type strain may be two or less, or four or more. In addition, the reference data do not need to be generated by mixing the molecules derived from a wild-type strain and the molecule derived from an in vitro transcript; for example, a mixture of a plurality of series data from the molecules derived from a wild-type strain may be used instead. In addition, in place of and/or in addition to the molecules derived from deletion strains, molecules derived from a mutant strain in which an enzyme involved in modification at a specific position that is not modified in the wild-type strain has been added may be used in the analysis.

[Diagnostic support method]

The analysis system 1 according to the present embodiment can be used in a method for supporting the diagnosis of a disease that causes a disorder in a modification state of a constituent unit in a molecule in which a plurality of constituent units of one or more types are bonded together.

The diagnostic support method according to the present embodiment includes: analyzing a specimen derived from a patient and containing a molecule in which a plurality of constituent units of one or more types are bonded together, and a specimen derived from a healthy subject and containing the molecule using the analysis system 1 according to the present embodiment; and analyzing a difference in the modification state of the constituent units in the molecule between the specimen derived from the patient and the specimen derived from the healthy subject.

In the diagnostic support method according to the present embodiment, for example, an attention map may be created based on the series data obtained from the patient-derived specimen and the series data obtained from the healthy subject-derived specimen. According to this aspect, it is possible to support the easy determination of whether there is a disorder in the modification state of the constituent units of the molecule in the patient.

In the diagnostic support method according to the present embodiment, for example, by analyzing the patient-derived specimen and the healthy subject-derived specimen using the same method as in the analysis example described with reference to Figure 6, it is also possible to estimate how the modification state of the molecule contained in the patient-derived specimen differs from that of the healthy subject-derived specimen.

[Additional note]

The present invention includes the following embodiment.

[1] A method for analyzing a modification state of a constituent unit in a molecule in which a plurality of constituent units of one or more types are bonded together, the method including: acquiring series data of signal values that vary in accordance with a type and a modification state of each constituent unit, the series data being obtained by scanning the molecule for each of a plurality of analyte molecules; aligning each of the plurality of series data, acquired from the plurality of analyte molecules, with reference data, thereby aligning the plurality of series data with each other; and analyzing a modification state of at least a part of the plurality of analyte molecules, based on the aligned plurality of series data.

[1-1] A method for analyzing a modification state of a base in a nucleic acid, the method including: acquiring series data of signal values that vary in accordance with a type and a modification state of each base, the series data being obtained by scanning the nucleic acid for each of a plurality of nucleic acids; aligning each of the plurality of series data, acquired from the plurality of nucleic acids, with reference data, thereby aligning the plurality of series data with each other; and analyzing a modification state of at least a part of the plurality of nucleic acids based on the aligned plurality of series data.
(Hereinafter, [1] and [1-1] will be collectively referred to as [1].)

[2] The analysis method according to [1], further including: generating the reference data by mixing a plurality of series data including the acquired series data.

[3] The analysis method according to [2], in which the plurality of series data to be mixed include two or more types of series data acquired or estimated from two or more types of molecules that have the same constituent unit sequence as that of at least one of the analyte molecules and that have different modification states of the constituent units from each other.

[4] The analysis method according to any one of [1] to [3], in which the analyzing of the modification states of the plurality of analyte molecules includes calculating a distance between a distribution of signal values at a predetermined point in the plurality of series data relating to a first molecule in which the modification state of the constituent units is a first modification state, and a distribution of signal values at a point of the plurality of series data relating to a second molecule in which the modification state of the constituent units is a second modification state, with the point being associated with the predetermined point, by using the plurality of series data acquired or estimated from the first molecule and aligned, and the plurality of series data acquired or estimated from the second molecule and aligned, and estimating a portion having a different modification state between the first molecule and the second molecule, based on the calculated distance.

[5] The analysis method according to any one of [1] to [4], in which the analyzing of the modification states of the plurality of analyte molecules includes estimating a modification state of a first constituent unit in a third molecule, in which the modification state of the first constituent unit is unknown, by using a machine learning model trained with training data, the training data including the series data acquired from a first molecule in which the modification state of the first constituent unit existing at a first position in a constituent unit sequence of the analyte molecules is a first modification state and the series data acquired from a second molecule in which the modification state of the first constituent unit is a second modification state.

[6] The analysis method according to any one of [1] to [5], in which the analyzing of the modification states of the plurality of analyte molecules includes classifying the aligned plurality of series data into two or more types of clusters by using an unsupervised machine learning model.

[7] The analysis method according to [6], in which the classifying into clusters includes calculating a similarity between the series data for each of the aligned plurality of series data, and classifying the aligned plurality of series data into two or more types of clusters based on the similarity.

[8] The analysis method according to any one of [1] to [7], in which the molecule is a nucleic acid.

[9] The analysis method according to [8], in which the plurality of analyte molecules include at least two types of: nucleic acids derived from a wild-type strain; nucleic acids having no modification; and nucleic acids derived from a mutant strain in which a modification state of a specific base has changed from that of the wild-type strain.

[9-1] The analysis method according to any one of [1] to [7], in which the plurality of analyte molecules include at least two types of: molecules derived from a wild-type strain; molecules having no modification; and molecules derived from a mutant strain in which a modification state of a specific base has changed from that of the wild-type strain. (Hereinafter, [9] and [9-1] will be collectively referred to as [9].)

[10] The analysis method according to [9], in which the plurality of analyte molecules include at least the nucleic acids derived from a wild-type strain, and a proportion of the number of bases having modifications relative to a total number of bases constituting the nucleic acids derived from a wild-type strain is 4% or more.

[10-1] The analysis method according to [9-1], in which the plurality of analyte molecules include at least the molecules derived from a wild-type strain, and a proportion of the number of constituent units having modifications relative to a total number of constituent units constituting the molecules derived from a wild-type strain is 4% or more. (Hereinafter, [10] and [10-1] will be collectively referred to as [10].)

[11] The analysis method according to any one of [1] to [10], further including: calculating redundancy of the series data relative to the reference data when aligning each of the plurality of series data with the reference data, in which the modification state is analyzed based on the calculated redundancy.

[12] A system for analyzing a modification state of a constituent unit in a molecule in which a plurality of constituent units of one or more types are bonded together, the system including: a measurement device configured to acquire series data of signal values that vary in accordance with a type and a modification state of each constituent unit, the series data being obtained by scanning the molecule for each of a plurality of analyte molecules; and an analysis device including an acquisition unit configured to acquire the series data acquired by the measurement device, an alignment unit configured to align each of the plurality of series data, acquired from the plurality of analyte molecules, with reference data, thereby aligning the plurality of series data with each other, and an analysis unit configured to analyze a modification state of at least a part of the plurality of analyte molecules, based on the aligned plurality of series data.

[13] An analysis device for analyzing a modification state of a constituent unit in a molecule in which a plurality of constituent units of one or more types are bonded together, the analysis device including: an acquisition unit configured to acquire series data of signal values that vary in accordance with a type and a modification state of each constituent unit, the series data being obtained by scanning the molecule for each of a plurality of analyte molecules from a measurement device configured to acquire the series data; an alignment unit configured to align each of the plurality of series data, acquired from the plurality of analyte molecules, with reference data, thereby aligning the plurality of series data with each other; and an analysis unit configured to analyze a modification state of at least a part of the plurality of analyte molecules, based on the aligned plurality of series data.

[14] A program for causing a computer to function as an analysis device for analyzing a modification state of a constituent unit in a molecule in which a plurality of constituent units of one or more types are bonded together, the program causing the computer to function as: an acquisition unit configured to acquire series data of signal values that vary in accordance with a type and a modification state of each constituent unit, the series data being obtained by scanning the molecule for each of a plurality of analyte molecules from a measurement device configured to acquire the series data; an alignment unit configured to align each of the plurality of series data, acquired from the plurality of analyte molecules, with reference data, thereby aligning the plurality of series data with each other, and an analysis unit configured to analyze a modification state of at least a part of the plurality of analyte moleculesm, based on the aligned plurality of series data.

[15] A method for assisting diagnosis of a disease that causes a disorder in a modification state of a constituent unit in a molecule in which a plurality of constituent units of one or more types are bonded together, the method including: analyzing a specimen containing the molecule and derived from a patient and a specimen containing the molecule and derived from a healthy subject, by the analysis method according to any one of [1] to [11]; and analyzing a difference in the modification state of the constituent units in the molecule between the specimen derived from the patient and the specimen derived from the healthy subject.

**[Example]**

Hereinafter, the present invention will be described in more detail with reference to an example. The present invention is not limited to the following example. In particular, in the following example, the modification state of tRNA is analyzed, but the analyte molecule is not limited to tRNA.

1. Nanopore sequencing of tRNA

(Overview)

For nanopore sequencing of an RNA sample, an adapter (2nd adapter) provided by Oxford nanopore technologies (ONT) was ligated (Figure 7(A)). A motor protein necessary for the RNA strand to pass through the nanopore is connected to the 2nd adapter. Due to differences in protruding sequences, instead of directly ligating the 2nd adapter to the tRNA, an adapter (1st adapter) was first ligated to mediate between the tRNA and the 2nd adapter. Thereafter, the tRNA ligated to the 1st adapter was purified by electrophoresis and gel excision, followed by further ligation with the 2nd adapter. Subsequent operations followed the Direct RNA sequencing manual published by ONT.

(Design of 1st adapter)

The 1st adapter was designed to consist of two adapters: a 3' adapter to be ligated to the 3' side of the tRNA and a 5' adapter to be ligated to the 5' side (Figure 7(B)). The last ten bases of the 3' adapter were designed as DNA, and the rest as RNA. To ligate to the tRNA, the adapter was designed to form a complementary strand to the protruding 3' terminal sequence of a mature tRNA molecule. Since five patterns (ACCA, UCCA, GCCA, CCCA, CCA) are conceivable for the protruding sequence at the 3' terminal of the mature tRNA depending on the tRNA species, 5' adapters corresponding thereto were designed as shown in the table below. In ligation with the 2nd adapter, the terminal DNA sequence of the 3' adapter was set to TAGTAGGTTC (SEQ ID NO: 1) so that the DNA sequence forms a complementary strand with the 2nd adapter. In addition, a spacer that does not participate in ligation but generates characteristic current values during nanopore sequencing was incorporated in the central portion of the 3' adapter. The spacer was used during data analysis, for example, for adapter trimming based on current signals.

[Table 1]

| Type | Sequence | SEQ ID NO |
|---|---|---|
| 5' adapter A | GGGAGGAUACUGGA | 2 |
| 5' adapter U | GGGAGGAUACUGGU | 3 |
| 5' adapter C | GGGAGGAUACUGGC | 4 |
| 5' adapter G | GGGAGGAUACUGGG | 5 |
| 5' adapter for His | GGGAGGAUACUGG | 6 |
| 3' adapter | GUAUCCUCCCAGAGAGAGAGAGAGAGA GAGAGAUUCCCCCtagtaggttc (lowercase letters indicate DNAs) | 7 |

(Ligation reaction of 1st adapter)

First, annealing of the 1st adapter was performed. A 5' adapter and a 3' adapter were mixed at a ratio of 3:2 and annealed at 75°C for 3 minutes. Thereafter, the temperature was decreased to 25°C at a rate of 1.2°C/min, and the adapter ligation reaction solution was mixed with the mixture.

[Table 2]

| Solution | Concentration |
|---|---|
| tRNA Sample | 1 $\mu$mol/L |
| 1st adapter solution | 10 mmol/L for 3'adapter |
| HEPES Buffer (pH:7.5) | 50 mmol/L |
| MgCl$_2$ | 2 mmol/L |
| Dithiothreitol (DTT) | 1 mmol/L |
| ATP | 0.9 mmol/L |
| PEG8000 | 5% |
| T4 RNA ligase 2 (NEB) (10unit/$\mu$L) | 0.5 unit/$\mu$L |

Thereafter, the reaction was carried out at 22°C for 6 hours. Subsequently, one-twentieth equivalent of 0.5 mol/L EDTA was added, one equivalent of PCI reagent was added, and the mixture was stirred. The mixture was centrifuged at 15000 g at 4°C for 10 minutes, and the upper layer was recovered. Thereafter, ethanol precipitation was performed, and dissolution in a purified form yielded tRNA ligated to the 1st adapter.

(Excision and purification of ligated tRNA)

A 7 M Urea 8% acrylamide gel was prepared and allowed to stand overnight. 10 $\mu$L of tRNA and 10 $\mu$L of 2$\times$ loading buffer were mixed to prepare a sample. After the sample solution was subjected to heat shock at 65°C for 5 minutes, the gel was set in an electrophoresis apparatus, and a pre-run was carried out at 100 V for 10 minutes. Wells were washed, and the sample and a marker were loaded. The marker was prepared by mixing 1 $\mu$L of DynaMarker RNA Low 2 with loading buffer. Electrophoresis was carried out at 100 V for 10 minutes and at 250 V for 40 minutes. The gel was removed, stained with toluidine blue, washed with purified water, and the portion between 100 nt and 200 nt containing the ligated tRNA was excised from the gel. The excised fragment was crushed in a 1.5 mL tube. 250 $\mu$L of Elution Buffer was added, stirred at 1100 rpm for 1 hour, and centrifuged to recover the supernatant. The addition of Elution Buffer, stirring, centrifugation, and recovery of the supernatant were repeated twice. The gel-containing solution was placed in a spin column and centrifuged at 12000 g at 4°C for 2 minutes. The filtrate was recovered and subjected to ethanol precipitation. Small molecules were removed by Drop Dialysis using a membrane filter, and the ligated tRNA was purified by dissolving in purified water.

(Ligation reaction of 2nd adapter)

As shown in the following table, a ligation reaction solution was mixed and allowed to react at 22°C for 20 minutes. One equivalent of RNA Clean XP was added and gently mixed for 5 minutes using a rotator. The beads were adsorbed with a magnet, the liquid was removed, 75 $\mu$L of WSB was added, and the mixture was tapped. After repeating this procedure, 21 $\mu$L of ELB was added instead of 75 $\mu$L of WSB, and the mixture was pipetted. After standing at room temperature for 10 minutes, the beads were adsorbed with a magnet and the liquid was recovered.

[Table 3]

| Solution | Used amount |
|---|---|
| Ligated tRNA sample | 10 pmol |
| RMX (2nd adapter) | 3 $\mu$L |
| 5x NEBNext Quick Ligation Buffer | 4 $\mu$L |
| T4 DNA ligase | 1.5 $\mu$L |
| Pure water | Up to 20 $\mu$L |

(Nanopore sequencing)

Nanopore sequencing was performed according to the Direct RNA sequencing manual published by ONT.

The reagents used in the above are listed below.

T4 RNA Ligase 2 (10 units/μL) (NEB M0239L)

DynaMarker RNA Low 2 (BioDynamics Laboratory Inc., DM152)

NEBNext Quick Ligation Reaction Buffer (NEB B6058)

T4 DNA Ligase (2,000 units/μL) (NEB M0202M)

RNA Clean XP (Beckman-Coulter A63987)

Direct RNA sequencing kit (Oxford nanopore technologies, SQK-RNA002)

Reagents having the following composition:

[Table 4]

(2x loading buffer for UreaPAGE)

| Reagent name | Concentration |
|---|---|
| Urea | 7 mol/L |
| Bromophenol blue | 0.05% |
| Xylene cyanol | 0.05% |

[Table 5]

**(Elution Buffer)**

| Reagent name | Concentration |
|---|---|
| NaOAc (pH:5.2) | 50 mmol/L |
| SDS | 0.1% |
| EDTA | 1 mmol/L |
| DTT | 1 mmol/L |

[Table 6]

**(Direct RNA sequencing kit (Oxford nanopore technologies SQK-RNA002))**

| Reagent name | Volume (μL) |
|---|---|
| RT Adapter | 10 |
| RNA Adapter Mix | 45 |
| RNA Calibrant Strand | 25 |
| Wash Buffer | 1200 |
| Elution Buffer | 300 |
| RNA Running Buffer | 600 |

2. Detection of modifications in *Escherichia coli* tRNA

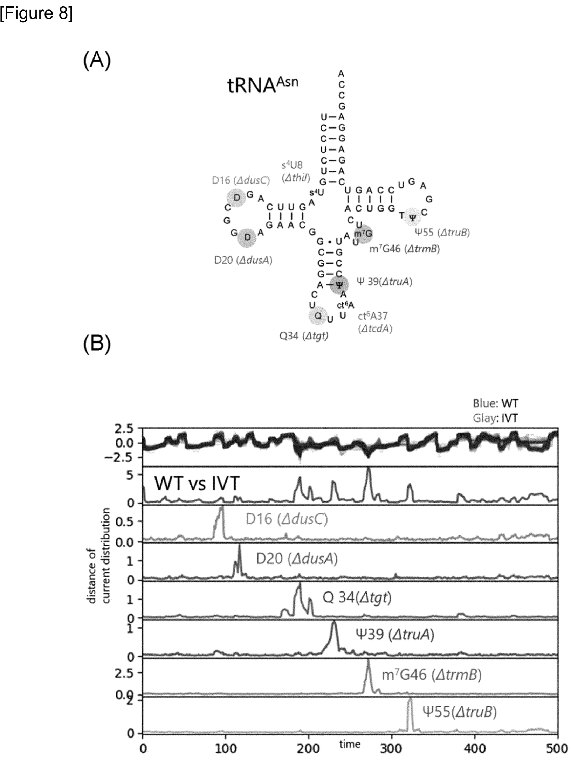

Detection of modifications in *Escherichia coli* tRNA[Asn] was performed. In *Escherichia coli* tRNA[Asn], as shown in Figure 8(A), seven types of RNA modifications are present at nine sites. tRNAs[Asn] derived from an *Escherichia coli* wild-type strain (hereinafter also referred to as "WT strain"), various tRNA modification enzyme deletion strains (hereinafter referred to as "KO strains"), and an in vitro transcript (hereinafter referred to as "IVT") were obtained or prepared, and current signals were measured by nanopore sequencing to obtain time-series data.

The time-series data derived from a WT strain and the time-series data derived from an IVT were mixed at a ratio of 1:1, reference data was created by DTW barycenter averaging, and all measured time-series data were aligned using this reference data. DTW was used for the alignment. When the time-series data derived from a WT strain and the time-series data derived from an IVT were compared with each other and the Kullback-Leibler (KL) distance was calculated for each point in the time-series data and plotted, a plurality of peaks were observed (the graph of WT vs. IVT in Figure 8(B)).

Next, when the time-series data derived from a WT strain and the time-series data derived from various KO strains were compared with each other and the Kullback-Leibler (KL) distance was calculated for each point in the time-series data and plotted, peaks were detected at positions corresponding to respective modification sites (Figure 8(B)).

The dihydrouridine at position 16 (D16) was detected as a peak in the comparison between the WT strain and the ΔdusC strain, and D20 was detected as a peak in the comparison between the WT strain and the ΔdusA strain. Similarly, with respect to each modification of queuosine at position 34 (Q34), pseudouridine at position 39 (Ψ39), 7-methylguanosine at position 46 (m$^7$G46), and Ψ55, peaks were detected in comparisons between the WT strain and the KO strains, such as the Δtgt strain, the ΔtruA strain, the ΔtrmB strain, and the ΔtruB strain, respectively. Since these KL distance peaks showed good agreement with the peaks observed in the comparison between the time-series data derived from a WT strain and the time-series data derived from an IVT, it was demonstrated that the analysis method according to the present embodiment enables the analysis of modification states in tRNA.

3. Detection of modifications on per-tRNA-molecule basis (Ψ55)

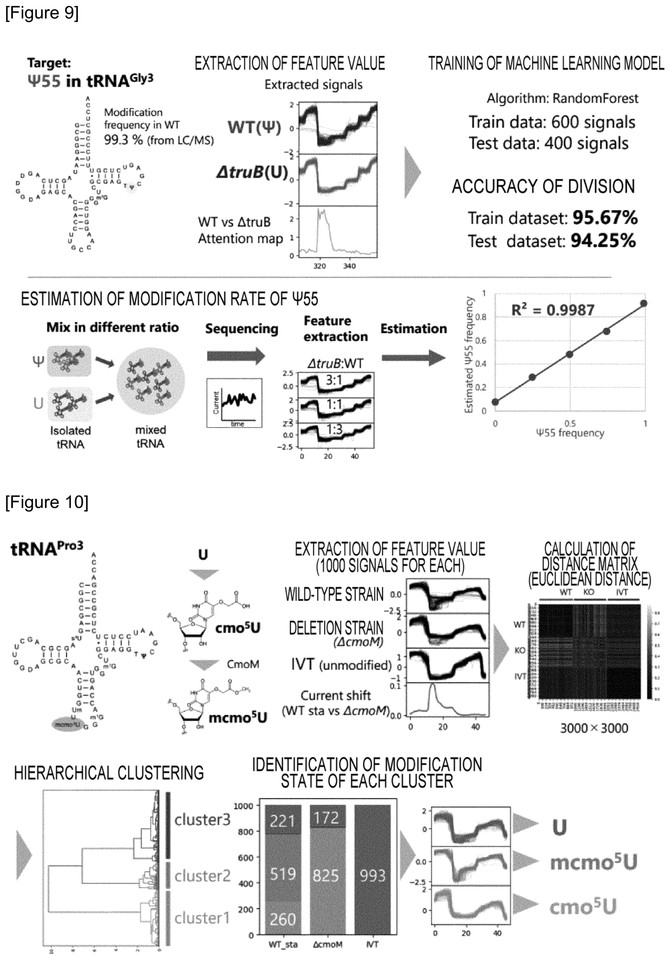

Next, an analysis using a supervised machine learning model was performed (Figure 9). As an analyte molecule, Ψ55 of *Escherichia coli* tRNA$^{Gly3}$ was selected. LC/MS analysis has revealed that, in the wild-type strain, this site is modified to Ψ at a rate of 99%or higher.

First, tRNAs$^{Gly3}$ derived from the WT strain, the ΔtruB strain, which is a KO strain, and an IVT were obtained or prepared. Then, electrical current signals were measured by nanopore sequencing to obtain time-series data. In the same manner as described in "2. Detection of modifications in *Escherichia coli* tRNA," the time-series data derived from the WT strain and the time-series data derived from the ΔtruB strain were compared with each other, and Kullback-Leibler (KL) distances were calculated at each point of the time-series data to create an attention map. The peak positions of the attention map were extracted as feature values, and a machine learning model (RandomForestClassifier (Breiman, L. Random Forests. Machine Learning 45, 5-32, doi:10.1023/a:1010933404324 (2001))) was trained using labeled training data, in which the time-series data derived from the WT strain were labeled as Ψ55 and the time-series data derived from the ΔtruB strain were labeled as unmodified uridine (U55). As a result, an accuracy of 94.25% was achieved for test data (upper section of Figure 9).

Furthermore, using a sample prepared by mixing tRNAs$^{Gly3}$ derived from the WT strain and the ΔtruB strain at appropriate ratios, time-series data were acquired by nanopore sequencing. Thereafter, the proportion of tRNA$^{Gly3}$ having Ψ55 in each sample was estimated using the above machine learning model. As a result, the actual mixing ratio of tRNA$^{Gly3}$ derived from the WT strain and tRNA$^{Gly3}$ derived from the ΔtruB strain showed good agreement with the proportion of tRNA$^{Gly3}$ having Ψ55 as estimated by the machine learning model (lower section of Figure 9). These results demonstrated that the analysis method according to the present embodiment enables highly accurate discrimination of modification states from individual time-series data.

4. Detection of modifications on per-tRNA-molecule basis (detection of plurality of modification states by clustering of electrical current signals)

Next, in a case where a plurality of modification states coexist, the modification states were classified by using an unsupervised machine learning model (Figure 10). As the analyte molecule, the modified uridine at position 34 of *Escherichia coli* tRNA$^{Pro3}$ was selected. It is known that three types of uridine, namely 5-methoxycarbonylmethoxyuridine (memo$^5$U34), 5-carboxymethoxyuridine (cmo$^5$U34), and unmodified uridine (U34), coexist at this position (Sakai, Y., Miyauchi, K., Kimura, S. & Suzuki, T. Biogenesis and growth phase-dependent alteration of 5-methoxycarbonylmethoxyuridine in tRNA anticodons. Nucleic Acids Res 44, 509-523, doi:10.1093/nar/gkv1470 (2016)).

First, in order to classify the above three types of modification states (mcmo$^5$U34, cmo$^5$U34, and U34) from time-series data derived from tRNA$^{Pro3}$ of the WT strain, tRNAs$^{Pro3}$ derived from the WT strain, the ΔcmoM strain, which is a KO strain, and an IVT were obtained or prepared. Then, electrical current signals were measured by nanopore sequencing to acquire time-series data. CmoM is an enzyme that methylates cmo$^5$U34 to form mcmo$^5$U34. In tRNA$^{Pro3}$ derived from the ΔcmoM strain, mcmo$^5$U34 is absent and only cmo$^5$U34 and U34 are present. On the other hand, only U34 is present in IVT tRNA$^{Pro3}$.

In the same manner as described in "2. Detection of modifications in *Escherichia coli* tRNA," the time-series data derived from the WT strain, the ΔcmoM strain, and the IVT were aligned. For all combinations of 1,000 time-series data each derived from the WT strain, ΔcmoM strain, and the IVT (3,000 data in total), the sum of distances between each point in two time-series data was calculated, and a 3,000 × 3,000 distance matrix was created. The calculated sum of distances corresponds to the similarity between the two time-series data, where a smaller sum of distances indicates a higher degree of similarity.

Hierarchical clustering using Ward's method was applied to the resulting distance matrix. When the data were divided into three clusters 1, 2, and 3, all of the time-series data derived from the IVT were classified into cluster 3; the time-series data derived from the ΔcmoM strain were classified into clusters 1 and 3; and the time-series data derived from the WT strain were classified into clusters 1, 2, and 3. Based on these results, it was estimated that cluster 3 was U34, cluster 2 was cmo⁵U34, and cluster 1 was mcmo⁵U34. The abundance ratios of the respective modification states in the tRNA derived from the WT strain were calculated based on the proportion of the number of time-series data contained in each cluster, and the results showed good agreement with the modification rates measured by LC/MS. Therefore, it was demonstrated that the analysis method according to the present embodiment enables detection and quantification of tRNA modifications.

### 5. Detection and quantification of taurine modification in human mitochondrial tRNA

It is known that mutations in human mitochondrial tRNA (mt-tRNA) genes cause mitochondrial diseases. A representative disease form, mitochondrial myopathy, encephalopathy, lactic acidosis, and stroke-like episodes (MELAS), is caused by a point mutation in the mt-tRNA$^{Leu(UUR)}$ gene (Goto Y, Nonaka I, Horai S (1990) A mutation in the tRNA(Leu)(UUR) gene associated with the MELAS subgroup of mitochondrial encephalomyopathies. Nature 348:651-653). It has also been reported that a significant decrease in the modification rate of 5-taurinomethyluridine (τm⁵U), a modification at position 34 in mutant mt-tRNA$^{Leu(UUR)}$ extracted from cells derived from MELAS patients, was observed (Suzuki T, Nagao A, Suzuki T (2011) Human mitochondrial tRNAs: biogenesis, function, structural aspects, and diseases. Annual review of genetics 45:299-329; Yasukawa T, Suzuki T, Suzuki T, Ueda T, Ohta S, Watanabe K (2000) Modification defect at anticodon wobble nucleotide of mitochondrial tRNAs(Leu)(UUR) with pathogenic mutations of mitochondrial myopathy, encephalopathy, lactic acidosis, and stroke-like episodes. J Biol Chem 275:4251-4257). The secondary structure of mt-tRNA$^{Leu(UUR)}$ is shown in Figure 12(A).

Therefore, it is considered that MELAS mutations (such as A3243G and T3271C) destabilize the three-dimensional structure of mt-tRNA$^{Leu(UUR)}$, thereby interfering with the recognition by tRNA modification enzymes and resulting in a decreased modification rate of τm⁵U34. Since τm⁵U34 is essential for decoding the UUG codon, the loss of this modification is considered to be one of the causes of mitochondrial dysfunction (above-cited Suzuki T, et al. (2011); Kirino Y, Yasukawa T, Ohta S, Akira S, Ishihara K, Watanabe K, Suzuki T (2004) Codon-specific translational defect caused by a wobble modification deficiency in mutant tRNA from a human mitochondrial disease. Proc Natl Acad Sci U S A 101:15070-15075).

Therefore, in this example, a model was created to classify the modification state of τm⁵U34 in mt-tRNA$^{Leu(UUR)}$, and it was demonstrated that the modification rate could be measured from data obtained by nanopore sequencing of total tRNA extracted from myoblasts derived from MELAS patients. First, mt-tRNA$^{Leu(UUR)}$ derived from HeLa cells, which served as the wild-type strain, and mt-tRNA$^{Leu(UUR)}$ derived from a τm⁵U modification-enzyme (GTPBP3) deletion strain were each used to measure electrical current signals by nanopore sequencing and acquire time-series data in accordance with the method described above.

In the same manner as described in "2. Detection of modifications in *Escherichia coli* tRNA," reference data were created based on the time-series data derived from HeLa cells and the time-series data from the modification-enzyme deletion strain. Then, using the reference data, all measured time-series data were aligned (Figure 12(B)). During the alignment, the Duration for each point in the time-series data was calculated based on the above Equations (I) to (IV) (Figure 12(B)).

In the same manner as described in "2. Detection of modifications in *Escherichia coli* tRNA," Kullback-Leibler (KL) distances of the time-series data of current values were calculated at each point based on all aligned time-series data derived from HeLa cells and all aligned time-series data derived from the modification-enzyme deletion strain (Figure 12(B)). Similarly, Kullback-Leibler (KL) distances were also calculated at each point of the time-series data of the Duration calculated above (Figure 12(B)).

As a result, peaks estimated to originate from τm⁵U34 were identified in the Kullback-Leibler (KL) distances of the current values and in the Kullback-Leibler (KL) distances of the Duration (indicated by a triangle in Figure 12(B)). At the respective peaks of the Kullback-Leibler (KL) distances of the current values and the Kullback-Leibler (KL) distances of the Duration, the current values and the Duration values were extracted, and when these values were plotted as a scatter diagram, it was confirmed that the distributions of the data derived from HeLa cells and the data derived from the modification-enzyme deletion strain were clearly separated (Figure 12(C)).

Based on the data in Figure 12(C), Gaussian distribution parameters for the distributions related to uridine (U) and τm⁵U on the scatter diagram of Duration × current value were determined using the expectation-maximization (EM) algorithm. Specifically, it was assumed that the current values and the Duration values for each modification state are distributed according to a single bivariate normal distribution, and a bivariate Gaussian mixture model was used. As a result, the modification rate of U derived from HeLa cells was estimated to be 91.2% (228 out of 250 reads were estimated to be τm⁵U), while the modification rate of U derived from the modification-enzyme deletion strain was estimated to be 1.7% (9 out of 541 reads were estimated to be τm⁵U) (Figure 12(D)). In Figure 12(D), **"*"** represents the center of the normal distribution, and the circle indicates the range of two standard deviations.

Furthermore, when this model was applied to the sequencing data derived from myoblasts of a MELAS patient, the modification rate was estimated to be 5.3% (4 out of 76 reads were estimated to be $\tau m^5U$) (Figure 12(D)). In this way, it was successfully demonstrated that the decrease in the taurine modification rate caused by the MELAS mutation could be measured through nanopore sequencing of human total tRNA.

6. Detection and quantification of multi-stage modification $mcm^5s^2U$ in yeast tRNA

In eukaryotic organisms such as humans and yeast, tRNAs corresponding to amino acids such as glutamic acid (Glu), lysine (Lys), and glutamine (Gln) have a modification of 5-methoxycarbonylmethyl-2- thiourdine ($mcm^5s^2U$) at the first position of the anticodon (position 34). $mcm^5s^2U$ Figure 13(A). $mcm^5s^2U$ Yoshida M, Kataoka N, Miyauchi K, Ohe K, Iida K, Yoshida S, Nojima T, Okuno Y, Onogi H, Usui T et al. (2015) Rectifier of aberrant mRNA splicing recovers tRNA modification in familial dysautonomia. Proc Natl Acad Sci U S A 112:2764-2769).

In addition, in humans, it has been reported that aberrant splicing of the ELP1 gene, which is essential for the biosynthesis of $mcm^5s^2U$, causes familial dysautonomia, and that abnormalities in the ELP3 gene are a cause of ALS, suggesting the importance of this modification (Anderson SL, Coli R, Daly IW, Kichula EA, Rork MJ, Volpi SA, Ekstein J, Rubin BY (2001) Familial dysautonomia is caused by mutations of the IKAP gene. Am J Hum Genet 68:753-758; Simpson CL, Lemmens R, Miskiewicz K, Broom WJ, Hansen VK, van Vught PW, Landers JE, Sapp P, Van Den Bosch L, Knight J et al. (2009) Variants of the elongator protein 3 (ELP3) gene are associated with motor neuron degeneration. Hum Mol Genet 18:472-481).

Therefore, in this example, a model was created for yeast tRNA$^{Gln1a}$ to classify three types of modifications, namely $mcm^5s^2U$, 5-methoxycarbonylmethyluridine ($mcm^5U$) which is one of its intermediates, and unmodified U.

First, tRNA$^{Gln1a}$ derived from a wild-type (WT) strain and tRNAs$^{Gln1a}$ derived from modification-enzyme deletion strains were prepared. As the deletion strains, an ncs6 deletion strain ($\Delta$ncs6) and a double deletion strain of elp3 and ncs6 ($\Delta$elp3$\Delta$ncs6) were used. Using these tRNAs, electric current signals were measured by nanopore sequencing and time-series data were acquired in accordance with the method described above.

The time-series data derived from a WT strain and the time-series data derived from $\Delta$elp3$\Delta$ncs6 were mixed at a ratio of 1:1, reference data was created by DTW barycenter averaging, and all measured time-series data were aligned using this reference data (Figure 13(B)). In addition, Duration was calculated at each point of the time-series data using the same method described in "5. Detection and quantification of taurine modification in human mitochondrial tRNA" (Figure 13(B)).

Then, using the same method described in "2. Detection of modifications in *Escherichia coli* tRNA," Kullback-Leibler (KL) distances were calculated at each point of the aligned time-series data of current values derived from $\Delta$elp3$\Delta$ncs6, based on the aligned time-series data of current values derived from the WT strain (Figure 13(B)). Similarly, the Kullback-Leibler (KL) distances were also calculated at each point of the time-series data of the Duration calculated above (Figure 13(B)). In addition, likewise, using the aligned time-series data of current values derived from the WT strain as a reference, the Kullback-Leibler (KL) distances were calculated for the aligned time-series data of current values derived from $\Delta$ncs6 and the aligned time-series data of Duration.

In both the two types of Kullback-Leibler (KL) distances calculated from the time-series data derived from $\Delta$elp3$\Delta$ncs6 and the two types of Kullback-Leibler (KL) distances calculated from the time-series data derived from $\Delta$ncs6, peaks were observed at nearby positions (Figure 13(B)). At the respective peaks of the Kullback-Leibler (KL) distances of the current values and the Kullback-Leibler (KL) distances of the Duration, the current values and the Duration values were extracted, and when these values were plotted as a scatter diagram, three types of distributions were confirmed among the data derived from the WT strain, the data derived from $\Delta$ncs6, and the data derived from $\Delta$elp3$\Delta$ncs6 (Figure 13(C)).

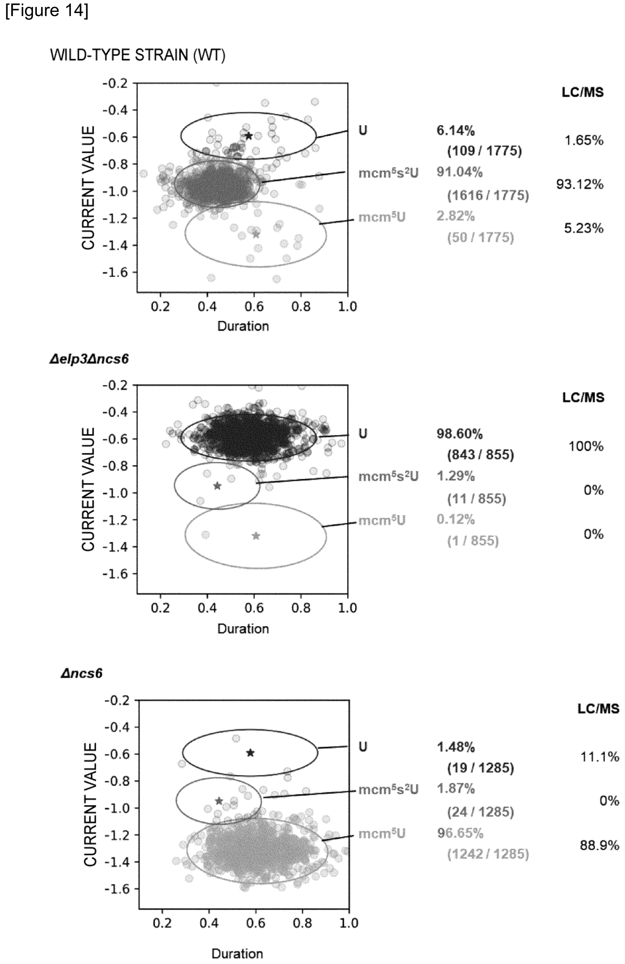

Based on the data in Figure 13(C), Gaussian distribution parameters for the distributions related to uridine (U), $mcm^5U$, and $mcm^5s^2U$ Figure 14). Specifically, $mcm^5s^2U$ $mcm^5U$ in $\Delta$ncs6. In Figure 14, "*" represents the center of the normal distribution, and the circle indicates the range of two standard deviations.

In this manner, it was demonstrated that the analysis method of the present embodiment enables detection of $mcm^5s^2U$, which is difficult to detect by conventional RNA sequencing. In addition, it was also demonstrated that $mcm^5U$ and U, which are present in trace amounts in the WT strain, could be detected.

Reference Signs List

1      Analysis system
10     Analysis device
10a    CPU
10b    RAM

10c     ROM
10d     Communication unit
10e     Input unit
10f     Display unit
11      Acquisition unit
12      Reference data generation unit
13      Alignment unit
14      Analysis unit
20      Measurement device

[Sequence Listing]

**Claims**

1.  A method for analyzing a modification state of a constituent unit in a molecule in which a plurality of constituent units of one or more types are bonded together, the method comprising:

    acquiring series data of signal values that vary in accordance with a type and a modification state of each constituent unit, the series data being obtained by scanning the molecule for each of a plurality of analyte molecules;
    aligning each of the plurality of series data, acquired from the plurality of analyte molecules, with reference data, thereby aligning the plurality of series data with each other; and
    analyzing a modification state of at least a part of the plurality of analyte molecules, based on the aligned plurality of series data.

2.  The analysis method according to claim 1, further comprising:
    generating the reference data by mixing a plurality of series data comprising the acquired series data.

3.  The analysis method according to claim 2, wherein
    the plurality of series data to be mixed comprise two or more types of series data acquired or estimated from two or more types of molecules that have the same constituent unit sequence as that of at least one of the analyte molecules and that have different modification states of the constituent units from each other.

4.  The analysis method according to claim 1, wherein

    the analyzing of the modification states of the plurality of analyte molecules comprises
    calculating a distance between a distribution of signal values at a predetermined point in the plurality of series data relating to a first molecule in which the modification state of the constituent units is a first modification state, and a distribution of signal values at a point of the plurality of series data relating to a second molecule in which the modification state of the constituent units is a second modification state, with the point being associated with the predetermined point, by using the plurality of series data acquired or estimated from the first molecule and aligned, and the plurality of series data acquired or estimated from the second molecule and aligned, and
    estimating a portion having a different modification state between the first molecule and the second molecule, based on the calculated distance.

5.  The analysis method according to claim 1, wherein

    the analyzing of the modification states of the plurality of analyte molecules comprises
    estimating a modification state of a first constituent unit in a third molecule, in which the modification state of the first constituent unit is unknown, by using a machine learning model trained with training data, the training data comprising the series data acquired from a first molecule in which the modification state of the first constituent unit existing at a first position in a constituent unit sequence of the analyte molecules is a first modification state and the series data acquired from a second molecule in which the modification state of the first constituent unit is a second modification state.

6.  The analysis method according to claim 1, wherein

the analyzing of the modification states of the plurality of analyte molecules comprises
classifying the aligned plurality of series data into two or more types of clusters by using an unsupervised machine learning model.

7. The analysis method according to claim 6, wherein

the classifying into clusters comprises
calculating a similarity between the series data for each of the aligned plurality of series data, and
classifying the aligned plurality of series data into two or more types of clusters, based on the similarity.

8. The analysis method according to claim 1, wherein
the molecule is a nucleic acid.

9. The analysis method according to claim 8, wherein
the plurality of analyte molecules comprise at least two types of: nucleic acids derived from a wild-type strain; nucleic acids having no modification; and nucleic acids derived from a mutant strain in which a modification state of a specific base has changed from that of the wild-type strain.

10. The analysis method according to claim 9, wherein

the plurality of analyte molecules comprise at least the nucleic acids derived from a wild-type strain, and
a proportion of the number of bases having modifications relative to a total number of bases constituting the nucleic acids derived from a wild-type strain is 4% or more.

11. The analysis method according to claim 1, further comprising:

calculating redundancy of the series data relative to the reference data when aligning each of the plurality of series data with the reference data, wherein
the modification state is analyzed based on the calculated redundancy.

12. A system for analyzing a modification state of a constituent unit in a molecule in which a plurality of constituent units of one or more types are bonded together, the system comprising:

a measurement device configured to acquire series data of signal values that vary in accordance with a type and a modification state of each constituent unit, the series data being obtained by scanning the molecule for each of a plurality of analyte molecules; and
an analysis device comprising
an acquisition unit configured to acquire the series data acquired by the measurement device,
an alignment unit configured to align each of the plurality of series data, acquired from the plurality of analyte molecules, with reference data, thereby aligning the plurality of series data with each other, and
an analysis unit configured to analyze a modification state of at least a part of the plurality of analyte molecules, based on the aligned plurality of series data.

13. An analysis device for analyzing a modification state of a constituent unit in a molecule in which a plurality of constituent units of one or more types are bonded together, the analysis device comprising:

an acquisition unit configured to acquire series data of signal values that vary in accordance with a type and a modification state of each constituent unit, the series data being obtained by scanning the molecule for each of a plurality of analyte molecules from a measurement device configured to acquire the series data;
an alignment unit configured to align each of the plurality of series data, acquired from the plurality of analyte molecules, with reference data, thereby aligning the plurality of series data with each other; and
an analysis unit configured to analyze a modification state of at least a part of the plurality of analyte molecules, based on the aligned plurality of series data.

14. A program for causing a computer to function as an analysis device for analyzing a modification state of a constituent unit in a molecule in which a plurality of constituent units of one or more types are bonded together, the program causing the computer to function as:

an acquisition unit configured to acquire series data of signal values that vary in accordance with a type and a modification state of each constituent unit, the series data being obtained by scanning the molecule for each of a plurality of analyte molecules from a measurement device configured to acquire the series data;

an alignment unit configured to align each of the plurality of series data, acquired from the plurality of analyte molecules, with reference data, thereby aligning the plurality of series data with each other, and

an analysis unit configured to analyze a modification state of at least a part of the plurality of analyte molecules, based on the aligned plurality of series data.

15. A method for assisting diagnosis of a disease that causes a disorder in a modification state of a constituent unit in a molecule in which a plurality of constituent units of one or more types are bonded together, the method comprising:

analyzing a specimen containing the molecule and derived from a patient and a specimen containing the molecule and derived from a healthy subject, by the analysis method according to any one of claims 1 to 11; and analyzing a difference in the modification state of the constituent units in the molecule between the specimen derived from the patient and the specimen derived from the healthy subject.

[Figure 1]

[Figure 2]

[Figure 3]

[Figure 4]

[Figure 5]

[Figure 6]

[Figure 7]

(A)

(B)

[Figure 8]

(A)

(B)

[Figure 9]

[Figure 10]

[Figure 11]

(A)

(B)

[Figure 12]

(A)

(B)

(C)

(D)

[Figure 13]

(A) Biosynthetic pathway of mcm⁵s²U

(B)

(C)

[Figure 14]

WILD-TYPE STRAIN (WT)

Δelp3Δncs6

Δncs6

## INTERNATIONAL SEARCH REPORT

International application No.

**PCT/JP2024/018330**

| A. | CLASSIFICATION OF SUBJECT MATTER |
|---|---|

*G01N 27/00*(2006.01)i; *C12Q 1/68*(2018.01)i
FI: G01N27/00 Z; C12Q1/68

According to International Patent Classification (IPC) or to both national classification and IPC

| B. | FIELDS SEARCHED |
|---|---|

Minimum documentation searched (classification system followed by classification symbols)

G01N27/00; C12Q1/68

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

JSTPlus/JMEDPlus/JST7580 (JDreamIII)

| C. | DOCUMENTS CONSIDERED TO BE RELEVANT |
|---|---|

| Category* | Citation of document, with indication, where appropriate, of the relevant passages | Relevant to claim No. |
|---|---|---|
| A | WO 2020/218489 A1 (OSAKA UNIVERSITY) 29 October 2020 (2020-10-29) | 1-15 |
| A | JP 8-237436 A (FUJI XEROX CO., LTD.) 13 September 1996 (1996-09-13) | 1-15 |
| A | US 2022/0328135 A1 (THE CHINESE UNIVERSITY OF HONG KONG) 13 October 2022 (2022-10-13) | 1-15 |

☐ Further documents are listed in the continuation of Box C. ☑ See patent family annex.

| | | |
|---|---|---|
| * | Special categories of cited documents: | "T" later document published after the international filing date or priority date and not in conflict with the application but cited to understand the principle or theory underlying the invention |
| "A" | document defining the general state of the art which is not considered to be of particular relevance | |
| "D" | document cited by the applicant in the international application | "X" document of particular relevance; the claimed invention cannot be considered novel or cannot be considered to involve an inventive step when the document is taken alone |
| "E" | earlier application or patent but published on or after the international filing date | |
| "L" | document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" | document referring to an oral disclosure, use, exhibition or other means | |
| "P" | document published prior to the international filing date but later than the priority date claimed | "&" document member of the same patent family |

| Date of the actual completion of the international search | Date of mailing of the international search report |
|---|---|
| **25 July 2024** | **06 August 2024** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | |
| | Telephone No. |

Form PCT/ISA/210 (second sheet) (July 2022)

## INTERNATIONAL SEARCH REPORT

| International application No. |
|---|
| **PCT/JP2024/018330** |

| **Box No. I** | **Nucleotide and/or amino acid sequence(s) (Continuation of item 1.c of the first sheet)** |
|---|---|

1. With regard to any nucleotide and/or amino acid sequence disclosed in the international application, the international search was carried out on the basis of a sequence listing:

    a. ☑ forming part of the international application as filed.

    b. ☐ furnished subsequent to the international filing date for the purposes of international search (Rule 13*ter*.1(a)),

        ☐ accompanied by a statement to the effect that the sequence listing does not go beyond the disclosure in the international application as filed.

2. ☐ With regard to any nucleotide and/or amino acid sequence disclosed in the international application, this report has been established to the extent that a meaningful search could be carried out without a WIPO Standard ST.26 compliant sequence listing.

3. Additional comments:

Form PCT/ISA/210 (continuation of first sheet) (July 2022)

## INTERNATIONAL SEARCH REPORT
### Information on patent family members

International application No.

**PCT/JP2024/018330**

| Patent document cited in search report | | | Publication date (day/month/year) | Patent family member(s) | | | Publication date (day/month/year) |
|---|---|---|---|---|---|---|---|
| WO | 2020/218489 | A1 | 29 October 2020 | US | 2023/0194472 | A1 | |
| JP | 8-237436 | A | 13 September 1996 | US | 5696610 | A | |
| US | 2022/0328135 | A1 | 13 October 2022 | JP | 2024-516365 | A | |
| | | | | WO | 2022/218290 | A1 | |
| | | | | EP | 4323539 | A1 | |
| | | | | CA | 3215066 | A1 | |
| | | | | CN | 117545855 | A | |
| | | | | KR | 10-2024-0007159 | A | |

Form PCT/ISA/210 (patent family annex) (July 2022)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **LIU, H. et al.** Accurate detection of m6A RNA modifications in native RNA sequences. *Nature communications*, 2019, vol. 10, 4079
- **STOIBER, M. et al.** De novo Identification of DNA Modifications Enabled by Genome-Guided Nanopore Signal Processing. *bioRxiv, 094672*, 2017
- **LEGER, A. et al.** RNA modifications detection by comparative Nanopore direct RNA sequencing. *Nature Communications*, 2021, vol. 12, 7198
- **SMITH, A. M. et al.** Reading canonical and modified nucleobases in 16S ribosomal RNA using nanopore native RNA sequencing. *PLoS One*, 2019, vol. 14, e0216709
- **THOMAS, N. K. et al.** Direct Nanopore Sequencing of Individual Full Length tRNA Strands. *ACS Nano*, 2021, vol. 15, 16642-16653
- **LUCAS, M. C. et al.** Quantitative analysis of tRNA abundance and modifications by nanopore RNA sequencing. *Nat Biotechnol*, 2023
- **BOCCALETTO, P. et al.** MODOMICS: a database of RNA modification pathways. 2021 update. *Nucleic Acids Res*, 2022, vol. 50, D231-D235
- **BREIMAN, L.** Random Forests. *Machine Learning*, 2001, vol. 45, 5-32
- **SAKAI, Y. ; MIYAUCHI, K. ; KIMURA, S. ; SUZUKI, T.** Biogenesis and growth phase-dependent alteration of 5-methoxycarbonylmethoxyuridine in tRNA anticodons. *Nucleic Acids Res*, 2016, vol. 44, 509-523
- **GOTO Y ; NONAKA I ; HORAI S.** A mutation in the tRNA(Leu)(UUR) gene associated with the MELAS subgroup of mitochondrial encephalomyopathies. *Nature*, 1990, vol. 348, 651-653

- **SUZUKI T ; NAGAO A ; SUZUKI T.** Human mitochondrial tRNAs: biogenesis, function, structural aspects, and diseases. *Annual review of genetics*, 2011, vol. 45, 299-329
- **YASUKAWA T ; SUZUKI T ; SUZUKI T ; UEDA T ; OHTA S ; WATANABE K.** Modification defect at anticodon wobble nucleotide of mitochondrial tRNAs(Leu)(UUR) with pathogenic mutations of mitochondrial myopathy, encephalopathy, lactic acidosis, and stroke-like episodes. *J Biol Chem*, 2000, vol. 275, 4251-4257
- **KIRINO Y ; YASUKAWA T ; OHTA S ; AKIRA S ; ISHIHARA K ; WATANABE K ; SUZUKI T.** Codon-specific translational defect caused by a wobble modification deficiency in mutant tRNA from a human mitochondrial disease. *Proc Natl Acad Sci U S A*, 2004, vol. 101, 15070-15075
- **YOSHIDA M ; KATAOKA N ; MIYAUCHI K ; OHE K ; LIDA K ; YOSHIDA S ; NOJIMA T ; OKUNO Y ; ONOGI H ; USUI T et al.** Rectifier of aberrant mRNA splicing recovers tRNA modification in familial dysautonomia. *Proc Natl Acad Sci U S A*, 2015, vol. 112, 2764-2769
- **ANDERSON SL ; COLI R ; DALY IW ; KICHULA EA ; RORK MJ ; VOLPI SA ; EKSTEIN J ; RUBIN BY.** Familial dysautonomia is caused by mutations of the IKAP gene. *Am J Hum Genet*, 2001, vol. 68, 753-758
- **SIMPSON CL ; LEMMENS R ; MISKIEWICZ K ; BROOM WJ ; HANSEN VK ; VAN VUGHT PW ; LANDERS JE ; SAPP P ; VAN DEN BOSCH L ; KNIGHT J et al.** Variants of the elongator protein 3 (ELP3) gene are associated with motor neuron degeneration. *Hum Mol Genet*, 2009, vol. 18, 472-481